# EUROPEAN PATENT SPECIFICATION

(11) **EP 1 180 039 B1**
(45) Date of publication and mention of the grant of the patent: **18.10.2006**
(21) Application number: 00918991.1
(22) Date of filing: 30.03.2000
(51) Int. Cl.: A61K 38/17, A61K 31/203, A61K 31/381, A61K 48/00, A61P 25/00

(54) **COMPOSITION FOR THE TREATMENT OF A NEUROLOGICAL DISORDER**
ZUBEREITUNG ZUR BEHANDLUNG VON EINER NEUROLOGISCHEN ERKRANKUNG
COMPOSITION POUR LE TRAITEMENT D'UNE MALADIE NEUROLOGIQUE

(30) Priority: 31.03.1999 GB 9907461
(43) Date of publication of application: 20.02.2002
(60) Divisional application: 06076367.9
(73) Proprietor: Oxford Biomedica (UK) Limited, Oxford 0X4 4GA (GB)
(72) Inventor: MADEN, Malcolm, 26/29 Drury Lane, London WC2B 5RL (GB); CORCORAN, Jonathan Patrick Thomas, 26/29 Drury Lane, London WC2B 5RL (GB)
(74) Representative: Harding, Charles Thomas
(86) International application number: PCT/GB2000/001211
(87) International publication number: WO 2000/057900

(56) References cited:
- WO-A-96/23070
- WO-A-97/02030
- WO-A-97/24116
- WO-A-99/21574
- US-A- 4 808 630
- JOHNSON A T ET AL: "IDENTIFICATION OF RETINOIC ACID RECEPTOR BETA SUBTYPE SPECIFIC AGONISTS" JOURNAL OF MEDICINAL CHEMISTRY,US,AMERICAN CHEMICAL SOCIETY. WASHINGTON, vol. 39, no. 26, 20 December 1996 (1996-12-20), pages 5027-5030, XP002065588 ISSN: 0022-2623
- MADEN M ET AL: "Retinoic acid as a chemotactic molecule in neuronal development." INTERNATIONAL JOURNAL OF DEVELOPMENTAL NEUROSCIENCE, vol. 16, no. 5, August 1998 (1998-08), pages 317-322, XP000946687 ISSN: 0736-5748 cited in the application
- MADEN M ET AL: "RETINOIC ACID AND DEVELOPMENT OF THE CENTRAL NERVOUS SYSTEM" BIOESSAYS, vol. 14, no. 7, 1992, pages 431-438, XP002149699 ISSN: 0265-9247
- HAN, GUANGYANG ET AL: "Enhanced potency of 9-cis versus all-trans-retinoic acid to induce the differentiation of human neuroblastoma cells" DIFFERENTIATION (BERLIN) (1995), VOLUME DATE 1995, 59(1), 61-9 , XP000946682
- CORCORAN JONATHAN ET AL: "Nerve growth factor acts via retinoic acid synthesis to stimulate neurite outgrowth." NATURE NEUROSCIENCE, vol. 2, no. 4, April 1999 (1999-04), pages 307-308, XP000946659 ISSN: 1097-6256 cited in the application
- CORCORAN JONATHAN ET AL: "The role of retinoic acid receptors in neurite outgrowth from different populations of embryonic mouse dorsal root ganglia." JOURNAL OF CELL SCIENCE, vol. 113, no. 14, July 2000 (2000-07), pages 2567-2574, XP000946689 ISSN: 0021-9533

## Description

### FIELD OF THE INVENTION

The present invention relates to a factor relating to neurite growth.

### BACKGROUND TO THE INVENTION

It is desirable to cause neurite development, such as neurite outgrowth and/or neurite regeneration, for example in cases of nervous injuries such as spinal cord injuries.

Nerve growth factor (NGF) is known to stimulate certain events such as neurite outgrowth. However, NGF is a relatively large molecule with a correspondingly high molecular weight. Moreover, NGF is susceptible to protease mediated degradation. Due to these and other considerations, NGF is difficult to administer. NGF is also relatively expensive to prepare. These are problems associated with the prior art.

### SUMMARY OF THE INVENTION

We have surprisingly found that it is possible to cause neurite development, such as neurite outgrowth and/or neurite regeneration, by using retinoic acid receptor β2 (RARβ2) and/or an agonist thereof.

### SUMMARY ASPECTS OF THE PRESENT INVENTION

The present invention is based on the surprising finding that it is possible to cause neurite development, such as neurite outgrowth and/or neurite regeneration, by using RARβ2 and/or an agonist thereof.

Aspects of the present invention utilise this finding. For example it is possible to have a method that causes modulation of neurite development, such as neurite outgrowth and/or neurite regeneration, by using RARβ2 and/or an agonist thereof as explained herein.

### DETAILED ASPECTS OF THE PRESENT INVENTION

In one aspect, the present invention relates to the use of RARβ2 and optionally an agonist thereof in the preparation of a medicament for the treatment of a neurological disorder.

In the present invention the RARβ2 and/or an agonist can be termed a pharmaceutically active agent.

Neurites are well known structures which develop from various neuronal cell types. They appear as microscopic branch or comb-like structures or morphological projections from the surface of the cell from which they emanate. Examples of neurite outgrowth are shown in the accompanying figures, and in publications such as those referenced in (Maden 1998-review article), and are well known in the art.

The RARβ2 coding sequence (i.e. the RARβ2 gene) is used as described hereinbelow. The RARβ2 gene may be prepared by use of recombinant DNA techniques and/or by synthetic techniques. For example, it may be prepared using the PCR amplified gene fragment prepared as in the Examples section of this document using the primers etc. detailed therein, or it may be prepared according to any other suitable method known in the art.

The agonist may, for example, be retinoic acid (RA) and/or CD2019.

Retinoic acid is commercially available. CD2019 is a polycyclic heterocarbyl molecule which is a RARβ2 agonist having the structure as discussed herein and as shown in (Elmazar *et al.,* (1996) Teratology vol. 53 pp158-167).

In another aspect, the present invention relates to the use of RARβ2 and optionally an agonist thereof in the preparation of a medicament for the treatment of a neurological disorder, wherein said neurological disorder is ameliorated by causing neurite development.

In another aspect, the present invention relates to the use of RARβ2 and optionally an agonist thereof in the preparation of a medicament for the treatment of a neurological disorder, wherein said neurological disorder comprises neurological injury.

The RARβ2 receptor may, for example, be administered by an entity comprising a RARβ2 expression system.

The RARβ2 expression system may comprise a nucleic acid construct capable of directing the expression of at least part of a RARβ2 receptor.

In order to determine whether an agent is capable of modulating RARβ2 signalling, one may use a method comprising providing neural cells, contacting said cells with said agent, and assessing the activity of the RARβ2 receptor, such as through the monitoring of neurite outgrowth.

Neural cells for use in the assay method may be any suitable neural cell line, whether stably maintained in culture, or primary cells derived from an animal directly. Preferably said cells will be embryonic mouse dorsal root ganglion (DRG) cells prepared as described hereinbelow.

If the agent is capable of modulating RARβ2 signalling in an *in vitro* assay method as described above, it may also be capable of affecting the *in vivo* activity of RARβ2.

In a further aspect, the invention relates to a pharmaceutical composition comprising RARβ2 and optionally an agonist thereof in admixture with a pharmaceutically acceptable carrier, diluent or excipient; wherein the pharmaceutical composition is for use in the treatment of a neurological disorder. For example, the pharmaceutical composition may cause neurite development.

For ease of reference, these and further aspects of the present invention are now discussed under appropriate section headings. However, the teachings under each section are not necessarily limited to each particular section.

### PREFERABLE ASPECTS

In a preferred aspect, the administration of a nucleic acid construct capable of directing the expression of RARβ2 will be accompanied by the administration of a RARβ2 agonist such as RA, or preferably CD2019 (or a mimetic thereof).

Preferably said agonist will be to some degree selective for the RARβ2 receptor. Preferably said agonist will not significantly affect the RARα receptor. Preferably said agonist will not significantly affect the RARγ receptor. More preferably said agonist will not significantly affect the RARα receptor or the RARγ receptor. Even more preferably, said agonist will exhibit a high degree of selectivity for the RARβ2 receptor.

In a preferred aspect, the administration of a nucleic acid construct capable of directing the expression of RARβ2 will be accomplished using a vector, preferably a viral vector, more preferably a retroviral vector. In a highly preferred embodiment, the administration of a nucleic acid construct capable of directing the expression of RARβ2 will be accomplished using a retroviral vector capable of infecting non-dividing mammalian cells such as neural cells.

### ADVANTAGES

The present invention is advantageous because RARβ2 and/or an agonist thereof can cause modulation of neural cell development.

It is also an advantage of the present invention that administration of NGF to a subject is avoided.

It is also an advantage of the present invention that it enable neurite outgrowth to be promoted in adult neural tissue.

### RETINOIDS

Retinoids are a family of molecules derived from vitamin A and include the biologically active metabolite, retinoic acid (RA). The cellular effects of RA are mediated through the action of two classes of receptors, the retinoic acid receptors (RARs) which are activated both by all-*trans*-RA (*t*RA) and 9*-cis-*RA (9-*cis*-RA), and the retinoid X receptors (RXRs), which are activated only by 9-*cis*-RA (Kastner et al., 1994; Kleiwer et al., 1994). The receptors are of three major subtypes, α, β and γ, of which there are multiple isoforms due to alternative splicing and differential promoter usage (Leid et al.). The RARs mediate gene expression by forming heterodimers with the RXRs, whilst the RXRs can mediate gene expression as homodimers or by forming heterodimers with a variety of orphan receptors (Mangelsdorf & Evans, 1995). Many studies on a variety of embryonic neuronal types have shown that RA can stimulate both neurite number and length (review, Maden, 1998), as, indeed, can the neurotrophins (Campenot, 1977; Lindsay, 1988; Tuttle and Mathew, 1995). The neurotrophins are a family of growth factors that are required for the survival of a variety of neurons of primary sensory neurons in the developing peripheral nervous system (Snider, 1994). One of the earliest genes induced by NGF in PC12 cells is the orphan receptor NGFI-B (NURR1) (Millbrandt, 1989). This suggests that the growth factor and retinoid mediated pathway in developing neurons can interact.

Background teachings on these aspects have been presented by Victor A. McKusick *et al* on http://www.ncbi.nlm.nih.gov/Omim. The following information has been extracted from that source.

Three retinoic acid receptors, alpha, beta, and gamma, are members of the nuclear receptor superfamily. Retinoic acid was the first morphogen described in vertebrates. The RARA and RARB genes are more homologous to those of the 2 closely related thyroid hormone receptors THRA and THRB, located on chromosomes 17 and 3, respectively, than to any other members of the nuclear receptor family. These observations suggest that the thyroid hormone and retinoic acid receptors evolved by gene, and possibly chromosome, duplications from a common ancestor which itself diverged rather early in evolution from the common ancestor of the steroid receptor group of the family. The RARB gene, formerly symbolized HAP, maps to 3p24 by somatic cell hybridization and in situ hybridization.
Benbrook et al. (1988) showed a predominant distribution in epithelial tissues and therefore used the designation RAR(epsilon). By in situ hybridization, Mattei et al. (1988) assigned the RARB gene to 3p24. Using deletion mapping, de The et al. (1990) identified a 27-bp fragment located 59-bp upstream of the transcriptional start, which confers retinoic acid responsiveness on the herpesvirus thymidine kinase promoter. They found indications that both alpha and beta receptors act through the same DNA sequence. Mattei et al. (1991) assigned the corresponding gene to chromosome 14, band A, in the mouse, and to chromosome 15 in the rat.
Nadeau et al. (1992) confirmed assignment of the mouse homolog to the centromeric portion of chromosome 14.
From a comparison of a hepatitis-B virus (HBV) integration site present in a particular human hepatocellular carcinoma (HCC) with the corresponding unoccupied site in the nontumorous tissue of the same liver, Dejean et al. (1986) found that HBV integration placed the viral sequence next to a liver cell sequence that bears a striking resemblance to both an oncogene, ERBA, and the supposed DNA-binding domain of the human glucocorticoid receptor and estrogen receptor genes.
Dejean et al. (1986) suggested that this gene, usually silent or transcribed at a very low level in normal hepatocytes, becomes inappropriately expressed as a consequence of HBV integration, thus contributing to the cell transformation.
By means of a panel of rodent-human somatic cell hybrid DNAs, Dejean et al. (1986) localized the gene to chromosome 3. Further studies by de The et al. (1987) suggested that the HAP gene product may be a novel ligand-responsive regulatory protein whose inappropriate expression in liver is related to hepatocellular carcinogenesis. Brand et al. (1988) showed that the novel protein called HAP (for HBV-activated protein) is a retinoic acid receptor. They referred to this receptor as the beta type (RARB) and mapped it to 3p25-p21.
Lotan et al. (1995) found that the expression of RARB mRNA is selectively lost in premalignant oral lesions and can be restored by treatment with isotretinoin. Restoration of the expression of RARB mRNA was associated with a clinical response.
RARB, RARG, RXRB, and RXRG are expressed in the striatum. To study the effect of these genes on locomotion, Kreczel et al. (1998) developed single and double knockout mice and analyzed their locomotor skills by open field and rotarod testing. RARB-RXRB, RARB-RXRG, and RXRB-RXRG double null mutant mice, but not the corresponding single null mutants, exhibited reductions in forward locomotion when compared with wildtype littermates. Forty percent of the RARB-RXRB null mutants showed backward locomotion. Rotarod test performance was impaired for RARB, RARB-RXRB, RARB-RXRG, and RXRB-RXRG mice. In contrast, RARA, RARG, RARA-RXRG, and RARG-RXRG null mice showed no defects in locomotion, even though both RARA and RARG are also expressed in the striatum. The morphology, development, and function of skeletal muscle, peripheral nerves, and spinal cord were normal in all single and double null mutants, as were balance reflexes. These results suggested to Kreczel et al. (1998) that RARB, RXRB, and RXRG are involved specifically in the control of locomotor behaviors, and that heterodimers of RARB with either RXRB or RXRG are the functional receptor units, such that RXRB and RXRG are functionally redundant.
Kreczel et al. (1998)studied the expression of D 1 and D2 dopamine receptors (D1R and D2R), the most abundant dopamine receptors in the striatum, in these mutant mice. RARB-RXRB, RARB-RXRG, and RXRB-RXRG double null mutants, but not RARB or RXRG single mutants, exhibited 40% and 30% reduction in whole-striatal D1R and D2R transcripts, respectively, when compared with wildtype controls.
The reduction was mostly in the medioventral regions of the striatum, including the shell and core of the nucleus accumbens, and the mediodorsal part of the caudate putamen. The reduction was not due to loss of D2R-expressing neurons; no increase in apoptosis was noted. The histology of the striatum was normal.
The characterization of a retinoic acid response element in the D2R promoter by Samad et al. (1997) led Kreczel et al. (1998) to suggest that the reduction in D2R and D2R expression occurs on a transcriptional level. The RARB-RXRB, RARB-RXRG, and RXRB-RXRG double null mutants did not exhibit the normal increase in locomotion induced by cocaine, mimicking the phenotype of D1R-null mice.
Taken together, these results indicated to Kreczel et al. (1998) that retinoids are involved in controlling the function of the dopaminergic mesolimbic pathway and suggested that defects in retinoic acid signaling may contribute to neurological disorders.

### AGONISTS

The agonist optionally used with RARβ2 in accordance with the present invention may be any suitable RARβ2 agonist. Preferably, said agonist of RARβ2 is capable of activating RARβ2 in a transactivation assay.

The agonist may be an organic compound or other chemical. The agonist can be an amino acid sequence or a chemical derivative thereof, or a combination thereof. The agent may even be a nucleotide sequence - which may be a sense sequence or an anti-sense sequence. The agent may even be an antibody.

Typically, the agonist will be an organic compound. Typically the organic compound will comprise two or more hydrocarbyl groups. Here, the term "hydrocarbyl group" means a group comprising at least C and H and may optionally comprise one or more other suitable substituents. Examples of such substituents may include halo-, alkoxy-, nitro-, an alkyl group, a cyclic group etc. In addition to the possibility of the substituents being a cyclic group, a combination of substituents may form a cyclic group. If the hydrocarbyl group comprises more than one C then those carbons need not necessarily be linked to each other. For example, at least two of the carbons may be linked *via* a suitable element or group. Thus, the hydrocarbyl group may contain hetero atoms. Suitable hetero atoms will be apparent to those skilled in the art and include, for instance, sulphur, nitrogen and oxygen. For some applications, preferably the agent comprises at least one cyclic group. The cyclic group may be a polycyclic group, such as a non-fused polycyclic group. For some applications, the agonist comprises at least the one of said cyclic groups linked to another hydrocarbyl group.

### SPECIFIC AGONISTS

An example of a specific agonist which may be used with RARβ2 in accordance with the present invention is retinoic acid (RA). Both common forms of retinoic acid (either all-trans retinoic acid (tRA), or 9-cis-RA) are agonists of RARβ2.

CD2019 is a RARβ2 agonist having the structure as discussed herein and as shown in (Elmazar *et al.,* (1996) Teratology vol. 53 pp158-167). This and other agonists are also discussed in (Beard and Chandraratna p.194; Johnson *et al.,* 1996). The structure of CD2019 is presented as Formula I in the attached figures.

An alternative RARβ2 agonist is presented as Formula II in the attached figures.

The present invention also contemplates the use of mimetics or bioisosteres of the formulae of Formula I and/or Formula II.

Preferably the agonist useful according to the present invention is selective for RARβ2.

### ASSAY TO DETERMINE RARβ2 AGONISM

Examples of agonists which may be used according to the present invention may be identified and/or verified by using an assay to determine RARβ2 agonism.

For example, the procedure may comprise (i) detemining if a candidate agent is capable of acting as a RARβ2 agonist; (ii) if said candidate agent is capable of acting as a RARβ2 agonist then using said agent in the preparation of a medicament with RARβ2 for the treatment of a neurological disorder.

### ASSAY

Any one or more of appropriate targets - such as an amino acid sequence and/or nucleotide sequence - may be used for identifying an agent capable of modulating RARβ2 in any of a variety of drug screening techniques. The target employed in such a test may be free in solution, affixed to a solid support, borne on a cell surface, or located intracellularly. The abolition of target activity or the formation of binding complexes between the target and the agent being tested may be measured.

The assay may be a screen, whereby a number of agents are tested. For example, the assay method may be a high through put screen.

Techniques for drug screening may be based on the method described in Geysen, European Patent Application 84/03564, published on September 13, 1984. In summary, large numbers of different small peptide test compounds are synthesized on a solid substrate, such as plastic pins or some other surface. The peptide test compounds are reacted with a suitable target or fragment thereof and washed. Bound entities are then detected - such as by appropriately adapting methods well known in the art. A purified target can also be coated directly onto plates for use in a drug screening techniques. Alternatively, non-neutralising antibodies can be used to capture the peptide and immobilise it on a solid support.

This invention also contemplates the use of competitive drug screening assays in which neutralising antibodies capable of binding a target specifically compete with a test compound for binding to a target.

Another technique for screening provides for high throughput screening (HTS) of agents having suitable binding affinity to the substances and is based upon the method described in detail in WO-A-84/03564.

The assay methods may be suitable for both small and large-scale screening of test compounds as well as in quantitative assays.

The assay may utilise cells that display RARβ2 on their surface. These cells may be isolated from a subject possessing such cells. However, preferably, the cells are prepared by transfecting cells so that upon transfect those cells display on their surface RARβ2.

Another example of an assay that may be used is described in WO-A-9849271, which concerns an immortalised human terato-carcinoma CNS neuronal cell line, which is said to have a high level of neuronal differentiation and is useful in detecting compounds which bind to RARβ2.

### REPORTERS

A wide variety of reporters may be used in the assay methods (as well as screens) with preferred reporters providing conveniently detectable signals (eg. by spectroscopy). By way of example, a reporter gene may encode an enzyme which catalyses a reaction which alters light absorption properties.

Other protocols include enzyme-linked immunosorbent assay (ELISA), radioimmunoassay (RIA) and fluorescent activated cell sorting (FACS). A two-site, monoclonal-based immunoassay utilising monoclonal antibodies reactive to two non-interfering epitopes may even be used. These and other assays are described, among other places, in Hampton R et al (1990, Serological Methods, A Laboratory Manual, APS Press, St Paul MN) and Maddox DE et al (1983, J Exp Med 15 8:121 1).

Examples of reporter molecules include but are not limited to (galactosidase, invertase, green fluorescent protein, luciferase, chloramphenicol, acetyltransferase, (glucuronidase, exo-glucanase and glucoamylase. Alternatively, radiolabelled or fluorescent tag-labelled nucleotides can be incorporated into nascent transcripts which are then identified when bound to oligonucleotide probes.

By way of further examples, a number of companies such as Pharmacia Biotech (Piscataway, NJ), Promega (Madison, WI), and US Biochemical Corp (Cleveland, OH) supply commercial kits and protocols for assay procedures. Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3817837; US-A-3850752; US-A-3939350; US-A-3996345; US-A-4277437; US-A-4275149 and US-A-4366241.

### HOST CELLS

Polynucleotides for use in the present invention - such as for use as targets or for expressing targets or for use as the pharmaceutically active agent - may be introduced into host cells.

The term "host cell" - in relation to the present invention includes any cell that could comprise the polynucleotide sequence.

Here, polynucleotides may be introduced into prokaryotic cells or eukaryotic cells, for example yeast, insect or mammalian cells.

Polynucleotides may be introduced into suitable host cells using a variety of techniques known in the art, such as transfection, transformation and electroporation. Where polynucleotides are to be administered to animals, several techniques are known in the art, for example infection with recombinant viral vectors such as retroviruses, herpes simplex viruses and adenoviruses, direct injection of nucleic acids and biolistic transformation.

Thus, a further embodiment of the present invention provides host cells transformed or transfected with a polynucleotide that is or expresses the target. Preferably said polynucleotide is carried in a vector for the replication and expression of polynucleotides that are to be the target or are to express the target. The cells will be chosen to be compatible with the said vector and may for example be prokaryotic (for example bacterial), fungal, yeast or plant cells.

The gram negative bacterium *E. coli* is widely used as a host for heterologous gene expression. However, large amounts of heterologous protein tend to accumulate inside the cell. Subsequent purification of the desired protein from the bulk of E.coli intracellular proteins can sometimes be difficult.

In contrast to *E.coli,* bacteria from the genus *Bacillus* are very suitable as heterologous hosts because of their capability to secrete proteins into the culture medium. Other bacteria suitable as hosts are those from the genera Streptomyces and Pseudomonas.

Depending on the nature of the polynucleotide encoding the polypeptide, and/or the desirability for further processing of the expressed protein, eukaryotic hosts such as yeasts or other fungi may be preferred. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

Examples of suitable expression hosts are fungi such as Aspergillus species (such as those described in EP-A-0184438 and EP-A-0284603) and Trichoderma species; bacteria such as Bacillus species (such as those described in EP-A-0134048 and EP-A-0253455), Streptomyces species and Pseudomonas species; and yeasts such as Kluyveromyces species (such as those described in EP-A-0096430 and EP-A-0301670) and Saccharomyces species. By way of example, typical expression hosts may be selected from *Aspergillus niger, Aspergillus niger var. tubigenis, Aspergillus niger var. awamori, Aspergillus aculeatis, Aspergillus nidulans, Aspergillus orvzae, Trichoderma reesei, Bacillus subtilis, Bacillus licheniformis, Bacillus amyloliquefaciens, Kluyveromyces lactis and Saccharomyces cerevisiae.*

Polypeptides that are extensively modified may require correct processing to complete their function. In those instances, mammalian cell expression systems (such as HEK-293, CHO, HeLA) are required, and the polypeptides are expressed either intracellularly, on the cell membranes, or secreted in the culture media if preceded by an appropriate leader sequence.

The use of suitable host cells - such as yeast, fungal, plant and mammalian host cells - may provide for post-translational modifications (e.g. myristoylation, glycosylation, truncation, lipidation and tyrosine, serine or threonine phosphorylation) as may be needed to confer optimal biological activity on recombinant expression products.

### ORGANISM

The term "organism" in relation to the present invention includes any organism that could comprise the sequence according to the present invention and/or products obtained therefrom. Examples of organisms may include a fungus, yeast or a plant.

The term "transgenic organism" in relation to the present invention includes any non-human organism that comprises the target according to the present invention and/or products obtained.

### TRANSFORMATION OF HOST CELLS/HOST ORGANISMS

As indicated earlier, the host organism can be a prokaryotic or a eukaryotic organism. Examples of suitable prokaryotic hosts include *E. coli* and *Bacillus subtilis.* Teachings on the transformation of prokaryotic hosts is well documented in the art, for example see Sambrook *et al* (Molecular Cloning: A Laboratory Manual, 2nd edition, 1989, Cold Spring Harbor Laboratory Press) and Ausubel et al., Current Protocols in Molecular Biology (1995), John Wiley & Sons, Inc.

If a prokaryotic host is used then the nucleotide sequence may need to be suitably modified before transformation - such as by removal of introns.

In another embodiment the transgenic organism can be a yeast. In this regard, yeast have also been widely used as a vehicle for heterologous gene expression. The species *Saccharomyces cerevisiae* has a long history of industrial use, including its use for heterologous gene expression. Expression of heterologous genes in *Saccharomyces cerevisiae* has been reviewed by Goodey et al (1987, Yeast Biotechnology, D R Berry et al, eds, pp 401-429, Allen and Unwin, London) and by King et al (1989, Molecular and Cell Biology of Yeasts, E F Walton and G T Yarronton, eds, pp 107-133, Blackie, Glasgow).

For several reasons *Saccharomyces cerevisiae* is well suited for heterologous gene expression. First, it is non-pathogenic to humans and it is incapable of producing certain endotoxins. Second, it has a long history of safe use following centuries of commercial exploitation for various purposes. This has led to wide public acceptability. Third, the extensive commercial use and research devoted to the organism has resulted in a wealth of knowledge about the genetics and physiology as well as large-scale fermentation characteristics of *Saccharomyces cerevisiae.*

A review of the principles of heterologous gene expression in *Saccharomyces cerevisiae* and secretion of gene products is given by E Hinchcliffe E Kenny (1993, "Yeast as a vehicle for the expression of heterologous genes", Yeasts, Vol 5, Anthony H Rose and J Stuart Harrison, eds, 2nd edition, Academic Press Ltd.).

Several types of yeast vectors are available, including integrative vectors, which require recombination with the host genome for their maintenance, and autonomously replicating plasmid vectors.

In order to prepare the transgenic Saccharomyces, expression constructs are prepared by inserting the nucleotide sequence of the present invention into a construct designed for expression in yeast. Several types of constructs used for heterologous expression have been developed. The constructs contain a promoter active in yeast fused to the nucleotide sequence, usually a promoter of yeast origin, such as the GAL1 promoter, is used. Usually a signal sequence of yeast origin, such as the sequence encoding the SUC2 signal peptide, is used. A terminator active in yeast ends the expression system.

For the transformation of yeast several transformation protocols have been developed. For example, a transgenic Saccharomyces according to the present invention can be prepared by following the teachings of Hinnen et al (1978, Proceedings of the National Academy of Sciences of the USA 75, 1929); Beggs, J D (1978, Nature, London, 275, 104); and Ito, H et al (1983, J Bacteriology 153, 163-168).

The transformed yeast cells are selected using various selective markers. Among the markers used for transformation are a number of auxotrophic markers such as LEU2, HIS4 and TRP1, and dominant antibiotic resistance markers such as aminoglycoside antibiotic markers, eg G418.

Another host organism is a plant. The basic principle in the construction of genetically modified plants is to insert genetic information in the plant genome so as to obtain a stable maintenance of the inserted genetic material. Several techniques exist for inserting the genetic information, the two main principles being direct introduction of the genetic information and introduction of the genetic information by use of a vector system. A review of the general techniques may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

Further hosts suitable for the nucleotide sequence include higher eukaryotic cells, such as insect cells or vertebrate cells, particularly mammalian cells, including human cells, or nucleated cells from other multicellular organisms. In recent years propagation of vertebrate cells in culture (tissue culture) has become a routine procedure. Examples of useful mammalian host cell lines are epithelial or fibroblastic cell lines such as Chinese hamster ovary (CHO) cells, NIH 3T3 cells, HeLa cells or 293T cells.

The nucleotide sequence may be stably incorporated into host cells or may be transiently expressed using methods known in the art. By way of example, stably transfected mammalian cells may be prepared by transfecting cells with an expression vector having a selectable marker gene, and growing the transfected cells under conditions selective for cells expressing the marker gene. To prepare transient transfectants, mammalian cells are transfected with a reporter gene to monitor transfection efficiency.

To produce such stably or transiently transfected cells, the cells should be transfected with a sufficient amount of the nucleotide sequence. The precise amounts of the nucleotide sequence may be empirically determined and optimised for a particular cell and assay.

A host cell may be transformed with a nucleotide sequence that is to be the target or is to express the target. Host cells transformed with the nucleotide sequence may be cultured under conditions suitable for the expression of the encoded protein. The protein produced by a recombinant cell may be displayed on the surface of the cell. If desired, and as will be understood by those of skill in the art, expression vectors containing coding sequences can be designed with signal sequences which direct secretion of the coding sequences through a particular prokaryotic or eukaryotic cell membrane. Other recombinant constructions may join the coding sequence to nucleotide sequence encoding a polypeptide domain which will facilitate purification of soluble proteins (Kroll DJ et al (1993) DNA Cell Biol 12:441-53).

### RECEPTORS

The RARβ2 receptor as discussed herein includes mimetics, homologues, fragments and part or all of the entire gene product. Preferably the RARβ2 receptor as discussed herein refers to substantially the entire gene product.

### NEUROLOGICAL DISORDERS

The term neurological disorders as used herein may refer to any injury, whether mechanically (for example by trauma) or chemically induced (for example by neurotoxin(s), or by an regime of treatment having an immunosuppressant effect, whether by design, or as a side-effect), any neural pathology such as caused by viral infection or otherwise, any degenerative disorder, or other nerve tissue related disorder.

Examples of neurological disorders include conditions such as Parkinson's disease, Alzheimer's disease, senility, motor neurone disease, schizophrenia as well as other neural and/or neurodegenerative disorders. Other neural related disorders may include glaucoma or other cause of damage to the optic nerve, Bell's palsy or other forms of localised paralysis, neurally based impotence such as caused by nerve trauma following radical prostatectomy, or other complaints. Other disorders in which the invention may be useful include neuropathological effects of diabetes, AIDS neuropathy, leprosy etc.

The term neurological disorder refers to any disorder of a nervous system, whether the peripheral nervous system or the central nervous system (CNS), whether the sympathetic nervous system, or the parasympathetic nervous system, or whether affecting a subset or superset of different nerve types.

### NUCLEOTIDE OF INTEREST (NOI)

In accordance with the present invention, the NOI sequence may encode a peptide which peptide may be the pharmaceutically active agent - such as an RA receptor, preferably RARβ2, or an agonist thereof.

Such coding NOI sequences may be typically operatively linked to a suitable promoter capable of driving expression of the peptide, such as in one or more specific cell types.

In addition to the NOI or part thereof and the expression regulatory elements described herein, the delivery system may contain additional genetic elements for the efficient or regulated expression of the gene or genes, including promoters/enhancers, translation initiation signals, internal ribosome entry sites (IRES), splicing and polyadenylation signals.

The NOI or NOIs may be under the expression control of an expression regulatory element, usually a promoter or a promoter and enhancer. The enhancer and/or promoter may be preferentially active in neural cells, such that the NOI is preferentially expressed in the particular cells of interest, such as in nerve cells. Thus any significant biological effect or deleterious effect of the NOI on the individual being treated may be reduced or eliminated. The enhancer element or other elements conferring regulated expression may be present in multiple copies. Likewise, or in addition, the enhancer and/or promoter may be preferentially active in one or more specific cell types - such as neural cells for example post-mitotically terminally differentiated non-replicating cells such as neurons.

The term "promoter" is used in the normal sense of the art, e.g. an RNA polymerase binding site in the Jacob-Monod theory of gene expression.

The term "enhancer" includes a DNA sequence which binds to other protein components of the transcription initiation complex and thus facilitates the initiation of transcription directed by its associated promoter.

### EXPRESSION VECTOR

Preferably, the NOI (e.g. that encoding RARβ2 or part thereof) used in the method of the present invention is inserted into a vector which is operably linked to a control sequence that is capable of providing for the expression of the coding sequence by the host cell, i.e. the vector is an expression vector.

### TARGETED VECTOR

The term "targeted vector" refers to a vector whose ability to infect/transfect/transduce a cell or to be expressed in a host and/or target cell is restricted to certain cell types within the host organism, usually cells having a common or similar phenotype.

### DELIVERY

The delivery system for use in the present invention may be any suitable delivery system for delivering said NOI and providing said NOI is expressed *in vivo* to produce said associated peptide (e.g. RARβ2), which in turn provides the beneficial therapeutic effect.

The delivery system may be a viral delivery system. Viral delivery systems include but are not limited to adenovirus vector, an adeno-associated viral (AAV) vector, a herpes viral vector, retroviral vector, lentiviral vector, baculoviral vector. Alternatively, the delivery system may be a non-viral delivery system - such as by way of example DNA transfection methods of, for example, plasmids, chromosomes or artificial chromosomes. Here transfection includes a process using a non-viral vector to deliver a gene to a target mammalian cell. Typical transfection methods include electroporation, DNA biolistics, lipid-mediated transfection, compacted DNA-mediated transfection, liposomes, immunoliposomes, lipofectin, cationic agent-mediated, cationic facial amphiphiles (CFAs) (Nature Biotechnology 1996 14; 556), and combinations thereof.

Other examples of vectors include *ex vivo* delivery systems - which include but are not limted to DNA transfection methods such as electroporation, DNA biolistics, lipid-mediated transfection, compacted DNA-mediated transfection).

In a preferred aspect, the delivery system is a vector.

In a more preferred aspect, the delivery system is a viral delivery system - sometimes referred to as a viral vector.

### VECTORS

As it is well known in the art, a vector is a tool that allows or faciliates the transfer of an entity from one environment to another. By way of example, some vectors used in recombinant DNA techniques allow entities, such as a segment of DNA (such as a heterologous DNA segment, such as a heterologous cDNA segment), to be transferred into a target cell. Optionally, once within the target cell, the vector may then serve to maintain the heterologous DNA within the cell or may act as a unit of DNA replication. Examples of vectors used in recombinant DNA techniques include plasmids, chromosomes, artificial chromosomes or viruses.

The term "vector" includes expression vectors and/or transformation vectors.

The term "expression vector" means a construct capable of *in vivo* or *in vitro*/*ex vivo* expression.

The term "transformation vector" means a construct capable of being transferred from one species to another.

### VIRAL VECTORS

In the present invention, the NOI may be introduced into suitable host cells using a viral delivery system (a viral vector). A variety of viral techniques are known in the art, such as for example infection with recombinant viral vectors such as retroviruses, herpes simplex viruses and adenoviruses.

Suitable recombinant viral vectors include but are not limited to adenovirus vectors, adeno-associated viral (AAV) vectors, herpes-virus vectors, a retroviral vector, lentiviral vectors, baculoviral vectors, pox viral vectors or parvovirus vectors (see Kestler et al 1999 Human Gene Ther 10(10):1619-32). In the case of viral vectors, gene delivery is typically mediated by viral infection of a target cell.

### RETROVIRAL VECTORS

Examples of retroviruses include but are not limited to: murine leukemia virus (MLV), human immunodeficiency virus (HIV), equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV).

Preferred vectors for use in accordance with the present invention are recombinant viral vectors, in particular recombinant retroviral vectors (RRV) such as lentiviral vectors.

The term "recombinant retroviral vector" (RRV) refers to a vector with sufficient retroviral genetic information to allow packaging of an RNA genome, in the presence of packaging components, into a viral particle capable of infecting a target cell. Infection of the target cell includes reverse transcription and integration into the target cell genome. The RRV carries non-viral coding sequences which are to be delivered by the vector to the target cell. An RRV is incapable of independent replication to produce infectious retroviral particles within the final target cell. Usually the RRV lacks a functional *gag-pol* and/or env gene and/or other genes essential for replication.

A detailed list of retroviruses may be found in Coffin *et al* ("Retroviruses" 1997 Cold Spring Harbour Laboratory Press Eds: JM Coffin, SM Hughes, HE Varmus pp 758-763).

### NON-VIRAL DELIVERY

The pharmaceutically active agent (e.g. the RARβ2) may be administered using non-viral techniques.

By way of example, the pharmaceutically active agent may be delivered using peptide delivery. Peptide delivery uses domains or sequences from proteins capable of translocation through the plasma and/or nuclear membrane

Polypeptides of interest such as RARβ2 may be directly introduced to the cell by microinjection, or delivery using vesicles such as liposomes which are capable of fusing with the cell membrane. Viral fusogenic peptides may also be used to promote membrane fusion and delivery to the cytoplasm of the cell.

Preferably, the RARβ2 or fragment(s) thereof may be delivered into cells as protein fusions or conjugates with a protein capable of crossing the plasma membrane and/or the nuclear membrane. Preferably, the RARβ2 or fragment(s) thereof is fused or conjugated to a domain or sequence from such a protein responsible for the translocational activity. Preferred translocation domains and sequences include domains and sequences from the HIV-1-trans-activating protein (Tat), *Drosophila* Antennapedia homeodomain protein and the herpes simplex-1 virus VP22 protein.

Exogenously added HIV-1-trans-activating protein (Tat) can translocate through the plasma membrane and to reach the nucleus to transactivate the viral genome. Translocational activity has been identified in amino acids 37-72 (Fawell et al., 1994, *Proc. Natl. Acad. Sci. U.* S. A. 91, 664-668), 37-62 (Anderson et al., 1993, *Biochem. Biophys. Res. Commun.* 194, 876-884) and 49-58 (having the basic sequence RKKRRQRRR) of HIV-Tat. Vives et al. (1997), *J Biol Chem* 272, 16010-7 identified a sequence consisting of amino acids 48-60 (CGRKKRRQRRRPPQC), which appears to be important for translocation, nuclear localisation and trans-activation of cellular genes. The third helix of the *Drosophila* Antennapedia homeodomain protein has also been shown to possess similar properties (reviewed in Prochiantz, A., 1999, *Ann N Y Acad Sci,* 886, 172-9). The domain responsible for translocation in Antennapedia has been localised to a 16 amino acid long peptide rich in basic amino acids having the sequence RQIKIWFQNRRMKWKK (Derossi, et al., 1994, *J Biol Chem,* 269, 10444-50). This peptide has been used to direct biologically active substances to the cytoplasm and nucleus of cells in culture (Theodore, et al., 1995, *J. Neurosci* 15, 7158-7167). The VP22 tegument protein of herpes simplex virus is capable of intercellular transport, in which VP22 protein expressed in a subpopulation of cells spreads to other cells in the population (Elliot and O'Hare, 1997, *Cell* 88, 223-33). Fusion proteins consisting of GFP (Elliott and O'Hare, 1999, *Gene Ther* 6, 149-51), thymidine kinase protein (Diiber et al., 1999, *Gene Ther* 6, 12-21) or p53 (Phelan et al., 1998, *Nat Biotechnol* 16, 440-3) with VP22 have been targeted to cells in this manner. Any of the domains or sequences as set out above may be used to direct RARβ2 or fragment(s) thereof into cell(s). Any of the domains or sequences as set out above, or others identified as having translocational activity, may be used to direct the RARβ2 or fragment(s) thereof into a cell.

### PHARMACEUTICAL COMPOSITIONS

The present invention also provides a pharmaceutical composition comprising administering a therapeutically effective amount of the agent of the present invention RARβ2 and optionally an agonist thereof as discussed herein) and a pharmaceutically acceptable carrier, diluent or excipients (including combinations thereof).

The pharmaceutical composition may comprise two components - wherein a first component comprises RARβ2 and a second component which comprises the agonist thereof. The first and second component may be delivered sequentially, simultaneously or together, and even by different administration routes.

The pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier,

The pharmaceutical compositions may comprise as - or in addition to - the carrier, excipient or diluent any suitable binder(s), lubricant(s), suspending agent(s), coating agent(s), solubilising agent(s).

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

### PHARMACEUTICAL COMBINATIONS

The agent of the present invention may be administered with one or more other pharmaceutically active substances. By way of example, the present invention covers the simultaneous, or sequential treatments with an agent according to the present invention and one or more steroids, analgesics, antivirals or other pharmaceutically active substance(s).

It will be understood that these regimes include the administration of the substances sequentially, simultaneously or together.

### EXAMPLES

The present invention will now be described, by way of example only, in which reference will be made to the following figures:
Figure 1 (which is Figure 1 referred to in Example 1) shows a photograph.
Figure 2, (which is Figure 2 referred to in Example 1) shows barcharts and a photograph.
Figure 3, (which is Figure 1 referred to in Example 2) shows a photograph.
Figure 4, (which is Figure 2 referred to in Example 2) shows a photograph.
Figure 5, (which is Figure 3 referred to in Example 2) shows a photograph.
Figure 6, (which is Figure 4 referred to in Example 2) shows a photograph.
Figure 7, (which is Figure 5 referred to in Example 2) shows a photograph.
Figure 8, (which is Figure 6 referred to in Example 2) shows a barchart.

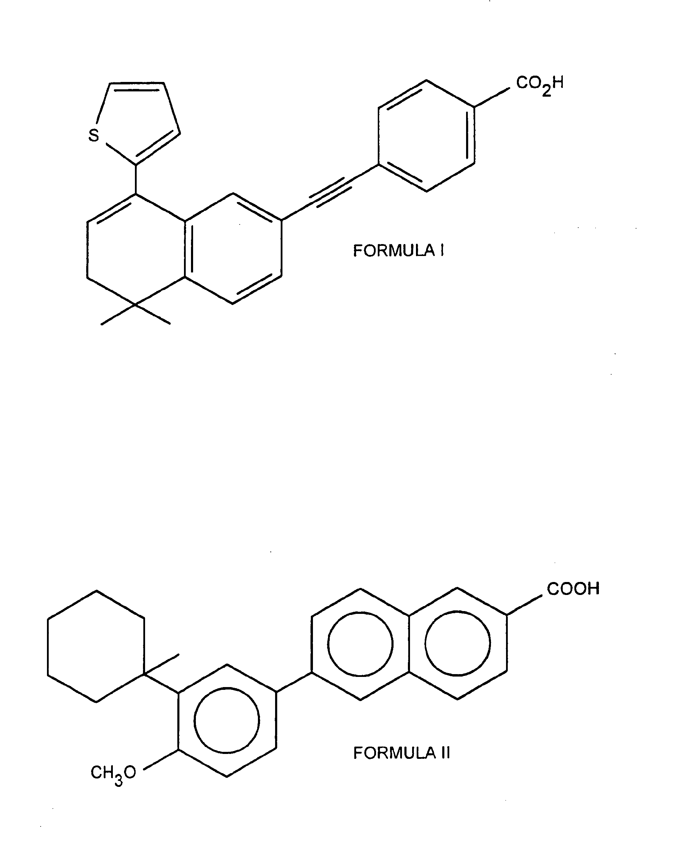

Figure 9, (which is Figure 1 referred to in Example 3) shows a photograph.
Figure 10, (which is Figure 2 referred to in Example 3) shows a photograph.
Figure 11, (which is Figure 3 referred to in Example 3) shows a photograph.
Figure 12, (which is Figure 4 referred to in Example 3) shows a photograph.
Figure 13, (which is Figure 5 referred to in Example 3) shows a photograph.
Figure 14, (which is Figure 6 referred to in Example 3) shows a photograph.
Figure 15, (which is Figure 7 referred to in Example 3) shows barcharts.
Figure 16, (which is Figure 8 referred to in Example 3) shows a photograph.
Figure 17 shows chemical formulae.

The figures are described more fully in the following example sections.

### EXAMPLE 1: STIMULATION OF NEURITE OUTGROWTH

Nerve growth factor acts via retinoic acid synthesis to stimulate neurite outgrowth.

Nerve growth factor (NGF) stimulates neurite outgrowth from cultured adult dorsal root ganglia (DRG)1. The vitamin A derivative retinoic acid (RA) also induces neurite outgrowth from various embryonic sources, including DRG2,3 Are such similarities in effects of NGF and RA because they are both components of the same genetic cascade leading to neurite outgrowth? RA up-regulates low-and high-affinity NGF receptors3,4 and induces the transcription of NGF itself5, suggesting that RA may be upstream of NGF in the cascade. However, here we show the converse, namely, that NGF is upstream of RA. We show that when adult mouse DRG are cultured in the presence of NGF and a compound that inhibits enzymes involved in RA synthesis, neurite outgrowth does not occur. Conversely, when RA is added along with a blocking antibody to NGF, neurite outgrowth occurs as normal. We further show that NGF induces transcription of both the retinoic acid-synthesizing enzyme RALDH-2 and the retinoic acid receptor-β as well as detectable release of synthesized RA. We propose that RA is required for adult DRG neurite regeneration and that NGF acts upstream of RA to induce its synthesis.

Cellular effects of RA are mediated by binding to nuclear receptors that are ligand activated transcription factors. There are two classes of receptors, retinoic acid receptors (RARs) and retinoid X receptors (RXRs), with three subtypes of each: α, β and γ6,7 In addition, there are multiple isoforms of each subtype due to alternative splicing and differential promoter usage. RAR receptors mediate gene expression by forming heterodimers with the RXRs, whereas RXRs can mediate gene expression either as homodimers or by forming heterodimers with orphan receptors such as LXR8 An additional mechanistic association between NGF and RA pathways is suggested by the findings that the nuclear receptor NGFIB heterodimerizes with the RXRs8 and that NGFIB is rapidly induced in PC 12 cells by the administration of NGF9.

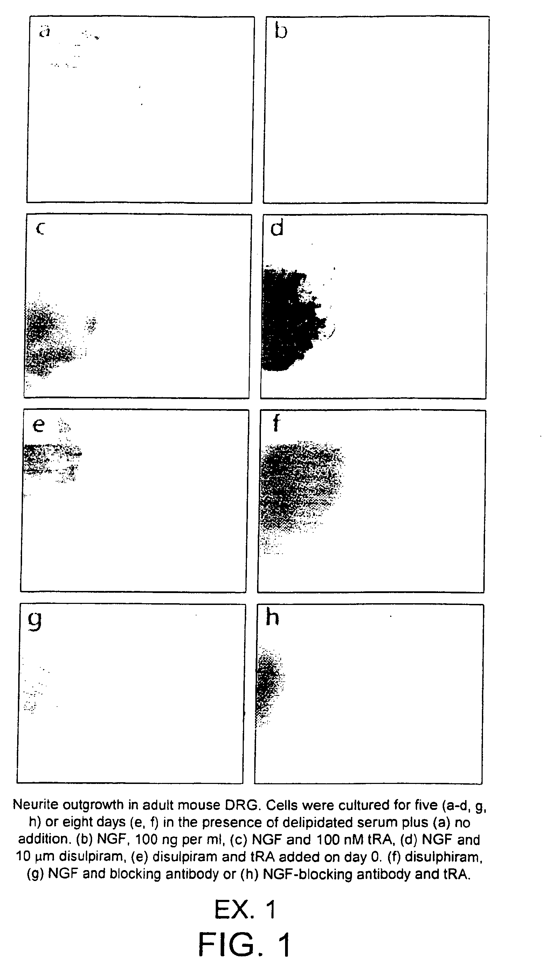

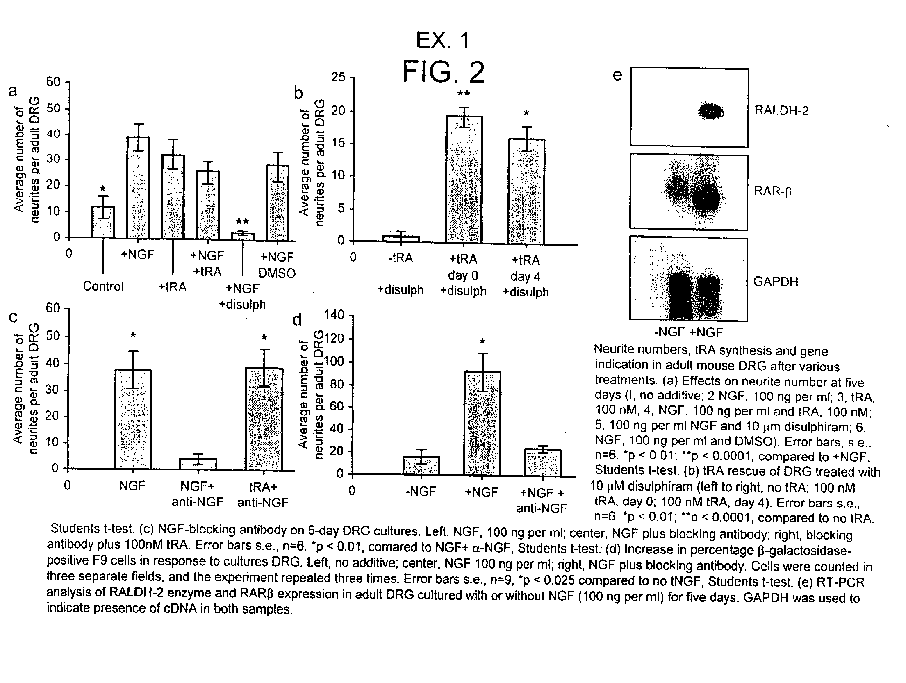

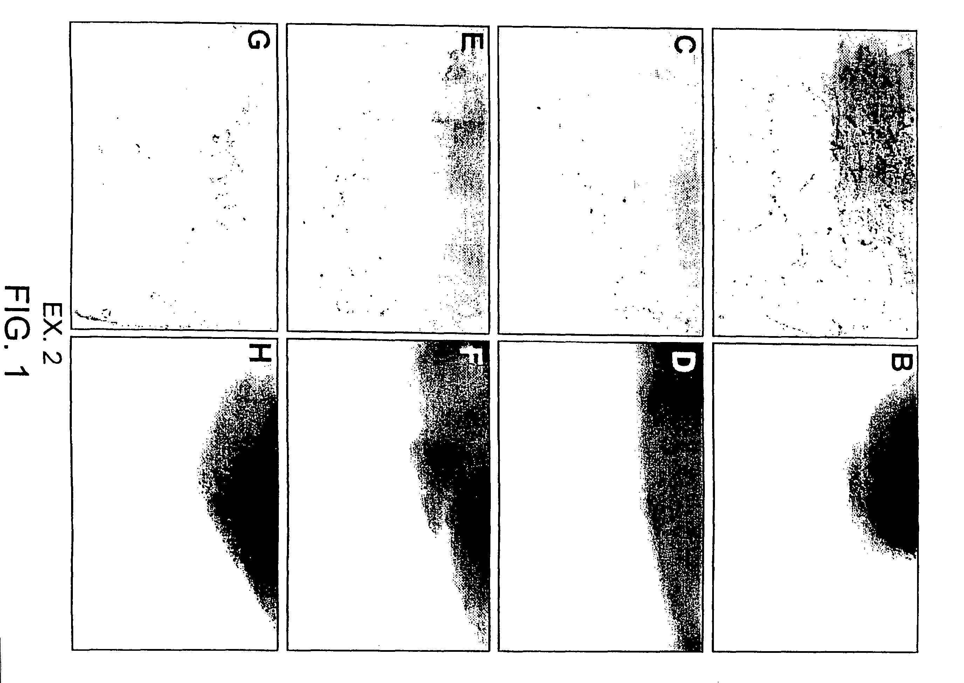

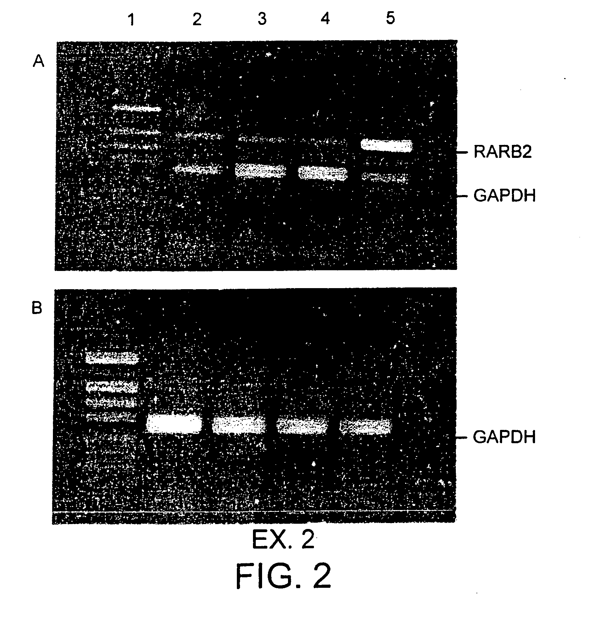

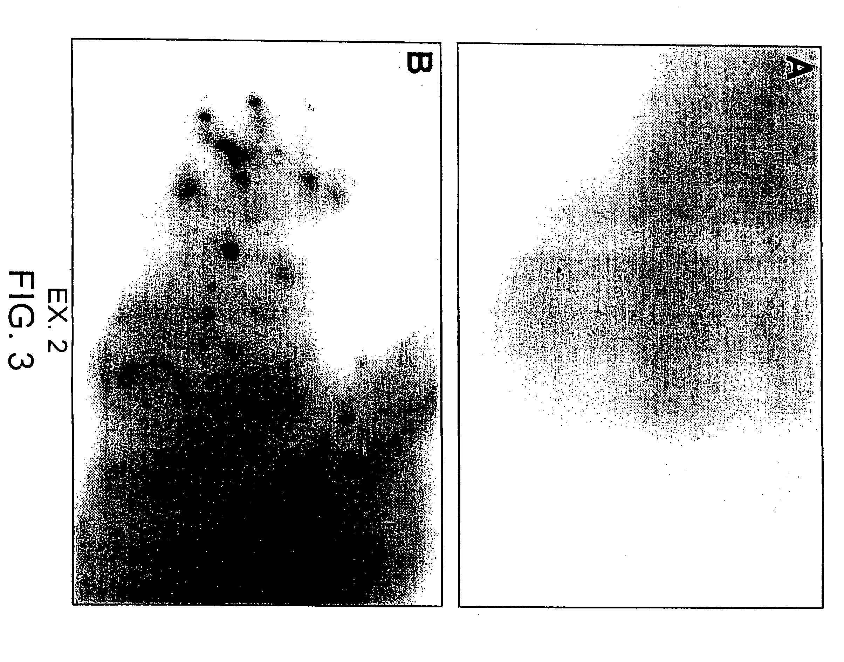

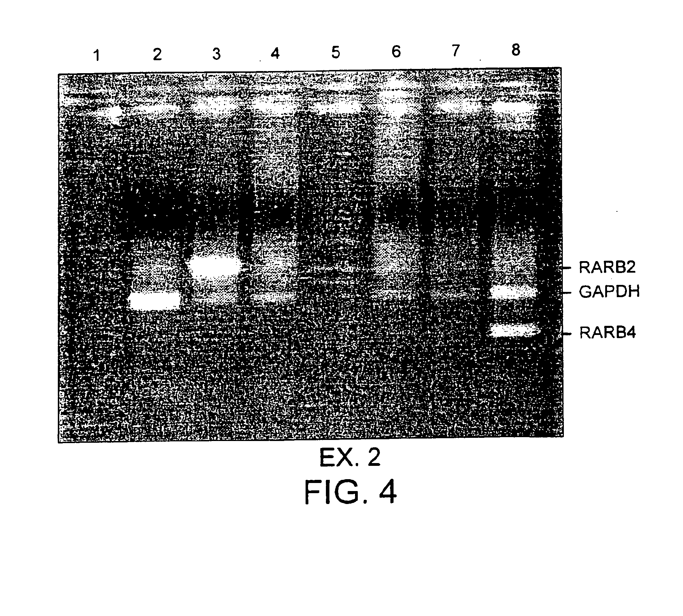

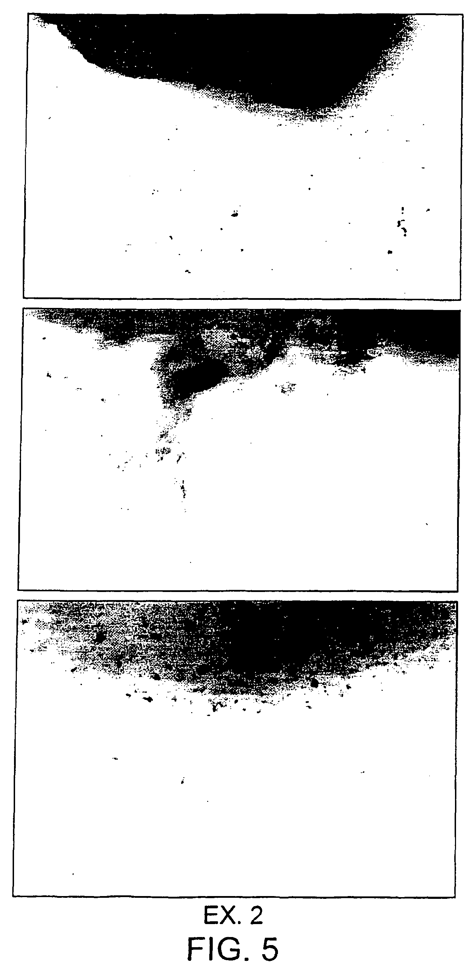

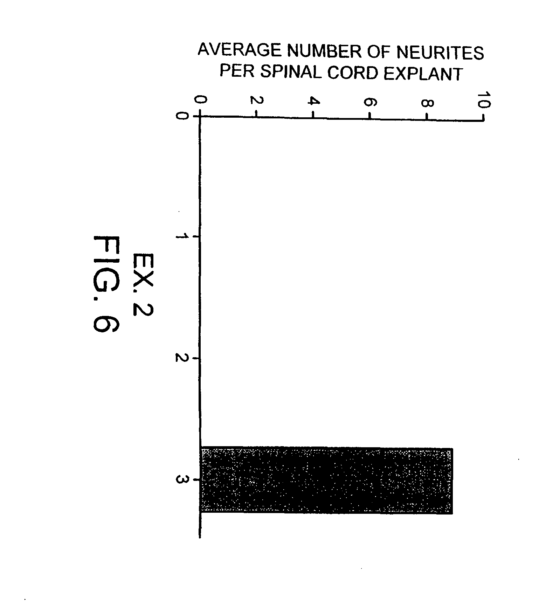

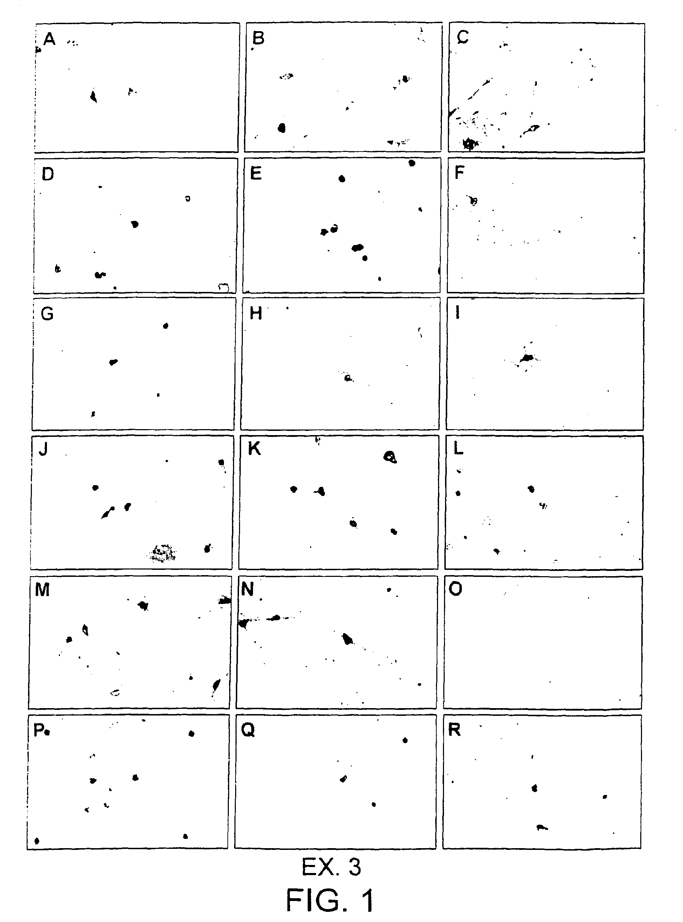

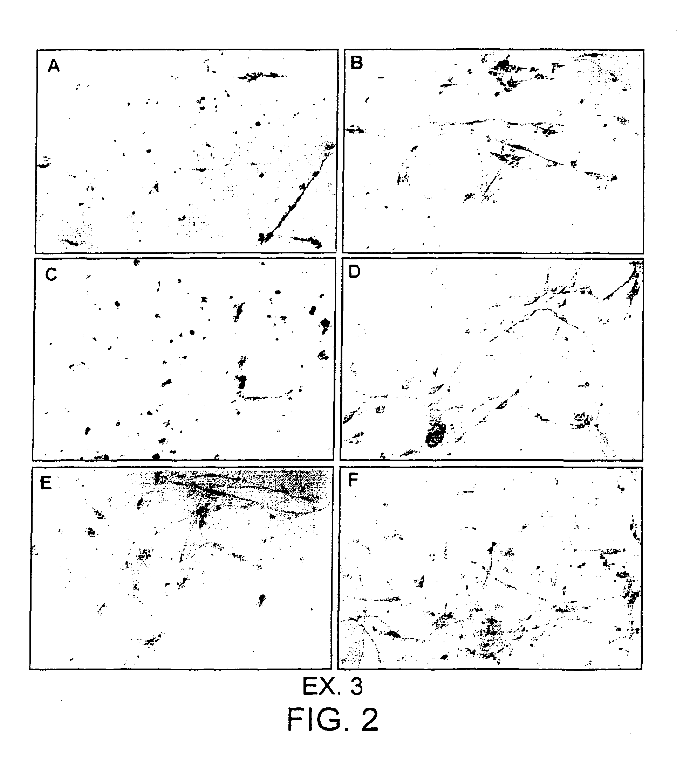

Although there is clearly a role for RA in the stimulation of neurite outgrowth from embryonic DRG2,3, it is not yet known if the same occurs in the adult DRG. To test this, we cultured adult mouse DRG in the presence of NGF (100 ng per ml), or RA (100 nM) in delipidated serum for five days. In both cases neurite outgrowth occurred **(Fig. 1b)** Little or no neurite outgrowth occurred in adult DRG cultured in only delipidated serum **(Fig. 1a).** Differences in number of neurites were significant **(Fig. 2a;** 1.2 and 3). It is important to note that the number of neurites extended from RA- or NGF-treated adult DRG, although significantly greater than the number extended from untreated DRG, was smaller than the number obtained using embryonic tissue. When RA was added together with NGF, there was no additive effect of the two treatments **(Fig. 1c)**, and no significant difference was seen between RA, NGF or RA plus NGF groups **(Fig. 2a**; 2, 3 and 4). Although it may be that both NGF and RA are at individual saturating concentrations, the lack of synergy may also imply that NGF and RA act through the same pathway in order to cause neurite outgrowth. One could imagine either RA inducing the production of NGF5, or NGF inducing the production of RA by stimulating a RA-synthesizing enzyme.

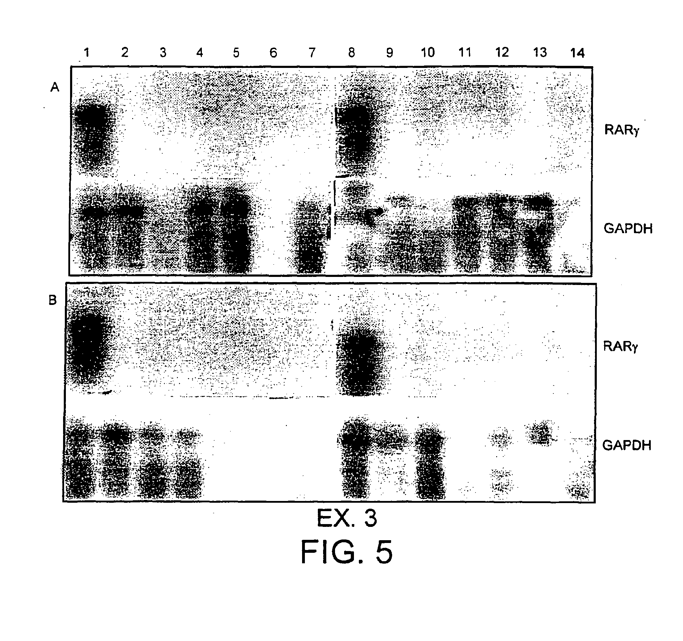

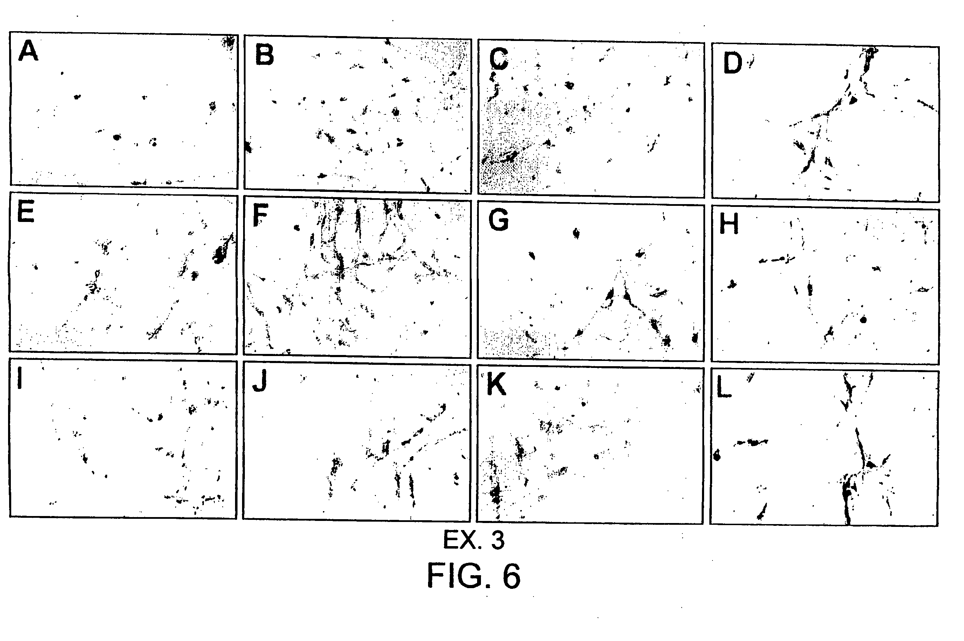

To test which of these hypotheses is most likely, we cultured adult DRG in the presence of NGF and 10 µM disulphiram, a compound which blocks the conversion of retinaldehyde to RA by inhibiting the enzyme aldehyde dehydrogenase 10. If RA acts to stimulate NGF production then disulphiram should have no effect on NGF-stimulated neurite outgrowth, whereas if NGF induces RA synthesis then disulphiram should inhibit outgrowth. As shown in Fig. 1d, addition of 10 µM disulphiram along with NGF completely abolished NGF-induced neurite outgrowth (significant difference; **Fig. 2a**, 2 and 5), whereas addition of DMSO (vehicle for disulphiram) and NGF did not affect neurite outgrowth (**Fig. 2a**, 2 and 6). To confirm that disulphiram did not affect cell survival within the explants, we performed two types of rescue. In both cases, explants were cultured for eight days in medium supplemented with disulphiram. In the first rescue, 100 nM RA was added to the explants from the beginning of the experiment; in the second, RA was added on day 4. In both cases, significantly greater neurite outgrowth occurred compared to cultures grown in medium supplemented with disulphiram alone (**Figs. 1e, f** and **2b**). These experiments also confirm the specificity of disulphiram for the RA synthesis pathway, as RA can rescue the cellular response.

Inhibition of the inductive effect of NGF but not of RA by disulphiram suggests that NGF may precede RA in the cascade leading to neurite outgrowth. To test this, we used a blocking antibody against NGF In the presence of NGF and the blocking antibody, virtually no neurite outgrowth occurred **(Fig. 1g;** compare to DRG cultured in the presence of NGF alone, **Fig. 1b).** On the other hand, DRG cultured in the presence of the NGF-blocking antibody and 100 nM RA **(Fig. 1h)** showed neurite outgrowth equivalent to that obtained with NGF alone **(Figs. 1b** and **2c**).

If NGF is upstream of RA, it should induce synthesis of RA after addition to DRG cultures. To test this prediction, we used an F9 reporter cell line that responds specifically to the presence of RA due to transfection with 1.8 kb of the mouse RARβ2 gene promoter containing a retinoic acid response element (RARE) linked to the *lacZ* gene (Sonneveld, E., van den Brink, C. E., van der Leede, B. J., Maden, M. & van der Saag, P. T. (1999) Embryonal carcinoma cell line stably transfected with mRARb2-lacZ: sensitive system for measuring levels of active retinoids. Exp.Cell.Res. vol. 250 pp284-297). In the presence of RA, activated cells can be detected after β-salactosidase histochemical staining. We first eliminated the possibility that NGF itself activates F9 cells by growing them in the presence of NGF (100 ng per ml), whereupon there was no labeling of the F9 cells above background. We then cultured adult DRG in delipidated serum for five days under three different conditions: in the absence of NGF, in the presence of NGF or in the presence of both NGF and the NGF-blocking antibody. Cultured DRG were then sonicated and placed on the F9 reporter cells. NGF-treated DRG homogenates produced a clear RA signal relative to untreated DRG (**Fig. 2d**). This activation was prevented when the DRG were cultured with blocking antibody in addition to NGF (**Fig. 2d**).

We next considered which retinoic acid synthesizing enzymes might be induced by NGF. Retinol is converted by a two-step oxidative process to an aldehyde, retinal, which is then oxidized to retinoic acid (for review, see ref. 11). It has been shown that retinaldehyde dehydrogenase type 2 (RALDH-2) is expressed in the developing nervous system, including the DRG12. Using RT-PCR, we found strong induction of RALDH-2 by NGF in cultured adult DRG as well (**Fig. 2e**). Lastly, we also found up-regulation of the RARβ receptor in NGF-stimulated cultures (**Fig. 2e**), a phenomenon shown to be involved in neurite outgrowth13.

Our results show that RA can stimulate neurite outgrowth from an adult neural tissue, the DRG. NGF similarly stimulates neurite outgrowth from this tissue, and we have demonstrated that it does so by inducing RA synthesis via an enzyme, RALDH-2. In the presence of either a NGF-blocking antibody or an inhibitor of RA synthesis, then NGF fails to act. Thus the most likely sequence of events in the induction of neurite outgrowth by NGF is: NGFRALDH-2RARARβ neurite outgrowth. We have not yet determined if NGF is directly responsible for inducing RALDH-2, or if some intermediary protein is required for this process. However, as NGFIB is one of the earliest genes induced by NGF9 and its product can heterodimerize with the RXRs8, the NGFIB/RXR heterodimer may be responsible for activating the RALDH-2 gene. Neurotrophins classically have been considered as potential agents for induction of nerve regeneration 14 and treatment of neurodegenerative diseases 15, but a major problem for their use is lack of effective modes of delivery to the site of the injury. Because RA is required for the regenerative response and it is downstream of NGF, then the problem of delivery to the lesion could be overcome, as RA is a low-molecular-weight lipophilic compound that can be administered orally. Thus, RA may be of clinical use in neurology.

### FIGURES FOR EXAMPLE 1

**Fig. 1**. Neurite outgrowth in adult mouse DRG cultured for five (**a-d, g, h**) or eight days (**e, f**) in the presence of delipidated serum plus: (a) no addition; (b) NGF, 100 ng per ml; (c) NGF and 100 nM tRA; (d) NGF and 10 M disulphiram; (e) disulphiram and tRA added on day 0; (f) disulphiram; (g) NGF and blocking antibody (h) NGF-blocking antibody and tRA.

**Fig. 2. (a-c)** Neurites produced by adult DRG cultured in cellogen. (a) Effects of NGF, RA and disulphiram at five days (**1**, no additive; **2**, NGF, 100 ng per ml: **3,** RA, 100 nM; **4**. NGF, 100 ng per ml and RA, 100 nM: **5**, 100 ng/ml NGF and 10 M disulphiram; **6**. NGF, 100 ng per ml and DMSO). Error bars, s.e.: n = 6, all groups. Differences between NGF-treated (2) and other groups: **p* < 0.01; ***p* < 0.0001: Student's *t*-test. (b) RA rescue of DRG treated with 10 M disulphiram (**left to right**: no RA; 100 nM RA, day 0; 100 nM RA, day 4) Error bars, s.e.; *n* = 6, all groups. Differences from RA-absent cultures: **p* < 0.01, ***p* < 0.0001; Student's *t*-test. (c) Effect of NGF-blocking antibody on 5-day DRG cultures. Left, NGF, 100 ng per ml; center, NGF plus blocking antibody; right, blocking antibody plus 100 nM RA; n = 4. Differences from NGF plus blocking antibody: **p* < 0.01, ***p* < 0.0001, Student's *t-*test. (**d**) Increase in percentage β-gal-positive F9 cells in response to DRG cultured with or without NGF. Left, no additive; center, NGF, 100 ng per ml; right, NGF with blocking antibody. Differences in percentage β-gal-positive cells from that produced by NGF-treated DRG ; **p* < 0.025, Student's *t*-test; for each group, *n* = 9. (**e**) RT-PCR analysis of RALDH-2 enzyme and RARβ expression in adult DRG cultured with or without NGF (100 ng per ml) for five days. GAPDH was used to indicate presence of cDNA in both samples. Use of F9 reporter cells in studying RA distribution in chick embryo has been described 16.

### EXAMPLE 2: INDUCTION OF NEURITE DEVELOPMENT IN ADULT NEURAL TISSUE.

It is surprisingly shown herein that retinoic acid receptor-β2 induces neurite outgrowth in the adult mouse spinal cord.

Retinoic acid has been shown to be required for neurite outgrowth. We have recently demonstrated that the mechanism of it's action in peripheral nerve regeneration is by activating the retinoic acid receptor β2. The adult central nervous system cannot regenerate. Therefore, we have investigated if regenerative failure in the adult spinal cord is related to the expression of retinoic acid receptor β2.

**Results:** We report here that in embryonic mouse spinal cord which can regenerate RARβ2 is up-regulated at concentrations which maximally stimulate neurite outgrowth. In contrast in the adult mouse spinal cord, RARβ2 is not detected nor is it induced by RA and no neurites are extended *in vitro.* When the adult cord is transfected with RARβ2 neurite regeneration can be induced. There is no neurite outgrowth when the cord is transfected with another isoform of RARβ, RARβ4. This shows the importance of receptor specificity in neurite regeneration.

**Conclusion:** These data suggest that the loss in regenerative potential of the adult CNS is due in part to the loss of expression of RARβ2 and that it is intrinsic to the neuron itself. We suggest that gene therapy with RARβ2 may result in functional recovery of the injured spinal cord.

**Background** The induction of axonal regeneration in the adult central nervous system (CNS) is a major goal in neurobiology. The failure of CNS axons to regenerate under normal circumstances has been attributed to one or a combination of causes: the low abundance of neurotrophic factors; the absence of growth-promoting molecules; the presence of growth-inhibiting molecules. Thus attempts to restimulate axon growth in the CNS have centered on these three possible. When peripheral nerve grafts were used to provide a permissive environment then spinal cord and medulla neurons extended axons up to 30mm in the adult rat¹. A similar strategy combined with fibroblast growth factor application resulted in the partial restoration of hind limb function². Neutralisation of neurite growth inhibitors present in myelin with antibodies permitted longer extension of axons than in control young rats³ and led to the recovery of specific reflex and locomotor functions after spinal cord injury⁴. A combination of neurotrophin-3 and these antibodies was successful in inducing long distance regeneration of corticospinal tract (CST) axons⁵. A suspension of olfactory ensheathing cells was also effective in returning locomotor function to the lesioned CST of rats⁶. If neurotrophins act simply to keep axotomised neurons alive⁷ then in these methodologies for inducing regeneration it is the environment surrounding the axons which is the focus of attention rather than the intrinsic capabilities of the neuron itself. However, at least part of the regenerative loss of the CNS is intrinsic to the neuron itself(4 refs). This suggests that the identification of genes that are not expressed in the non regenerating adult CNS but are in the developiong CNS which can regenerate neurites may lead to new stratagies of treatment of spinal cord injuries by gene therapy.

We show here that one such gene is RARβ2 which is activated by retinoic acid (RA) the biologically active metabolite of vitamin A. RA is present in various tissues of the developing embryo and adult animal, especially the nervous system⁸⁻¹³. In its absence, developing neurons of the CNS do not extend neurites into the periphery^{14,15}. Conversely, when applied to cultured neurons, RA induces both a greater number and longer neurites¹⁶ as well as being capable of dictating their direction of growth¹⁷. RA acts at the level of gene transcription because it is a ligand for two classes of nuclear transcription factors, the retinoic acid receptors (RARs) and the retinoid X receptors (RXRs)^{18,19}. There are three members of each class of retinoid receptor a, b and g as well as several isoforms of each member and this diversity may be responsible for the pleitropic effects of RA on cells.

We have been studying the molecular mechanisms of action of RA on neurons and have concluded that one of these retinoic acid receptors, RARβ2 is the crucial transducer of the RA signal in neurons as it is up-regulated in situations where RA stimulates neurite outgrowth²⁰. We hypothesised therefore that the absence or below threshold level of this nuclear receptor in the adult spinal cord may contribute to the failure of this tissue to regenerate axonal projections.

### Results and discussion

### Effect of RA on embryonic mouse spinal cord in vitro.

We began by confirming that the mouse embryonic spinal cord will respond to RA by extending neurites as do other areas of the embryonic CNS^{12,17, 21-23} and that this behaviour involves an up-regulation of RARβ2. E13.5 spinal cord was dissected from mouse embryos placed in a cellogen matrix and cultured in 10 % delipidated serum. All*-trans-*RA was added at 3 different concentrations (10⁻⁸M, 10⁻⁷M, 10⁻⁶M) and after 5 days the explants were stained with a neurofilament antibody and examined for the presence of neurites. There was an increasing number of neurites emerging from the cultured cord with increasing concentrations of RA with the maximal effect at 10⁻⁶M (Fig. 1C, E, G). Even in the absence of RA the embryonic cord extended neurites (Fig. 1A) presumably because of the high endogenous content of RA and its precursor retinol^{9,13}. Indeed, when the endogenous synthesis of RA is inhibited with disulphiram then no neurites are extended²⁴. To demonstrate that the induction of neurites involved the up-regulation of RARβ2, RT-PCR was performed on cultures after 5 days in the same range of RA treatments. This revealed that RARβ2 is normally expressed in embryonic spinal cord at all concentrations of RA used (Fig. 2A, lanes 1-5) and that it is strongly up-regulated after 1 x 10⁻⁶ M RA treatment (Fig. 2A, lane 5), the same concentration which gives maximal neurite outgrowth.

### Lack of effect of RA on adult mouse spinal cord in vitro.

We next performed an identical series of experiments using 10 month old adult spinal cord rather than the embryonic cord. In contrast to the embryonic cord, RA had no effect on neurite outgrowth at any concentration tested and like the untreated controls, these RA treated adult cords failed to extend any neurites at all (Fig. 1B, D, F, H). Examining the involvement of RARβ2 by RT-PCR revealed that control adult spinal cord had little or no detectable endogenous levels of this receptor (Fig. 2B, lane 1) and that there was no change in its level in response to RA treatment at any concentration (Fig. 2, lanes 2-5), unlike the embryonic cord.

### Induction of neurites in adult spinal cord

We therefore hypothesised that it was the lack of RARβ2 expression which may be responsible for the completely inert behaviour of the adult spinal cord. Our previous observations that adult DRG which do respond to RA by extending neurites also up-regulate RARβ2²⁴ demonstrates that the same behaviour is elicited by embryonic and the appropriate adult neurons and reinforces the differences in regulative behaviour between PNS and CNS neurons. To test our hypothesis we used a defective herpes simplex virus type 1 (HSV-1) vector to transfect pieces of adult (10 months) mouse spinal cord.

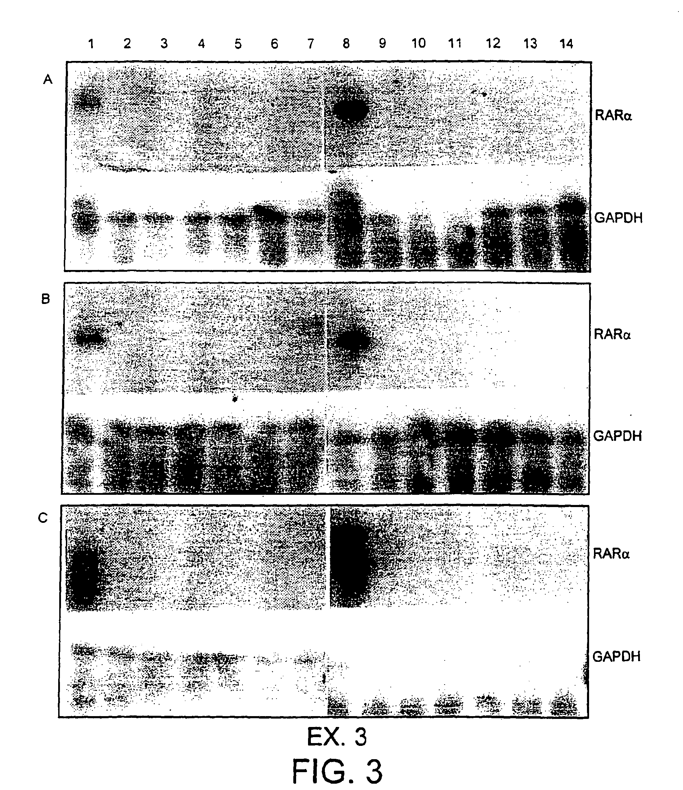

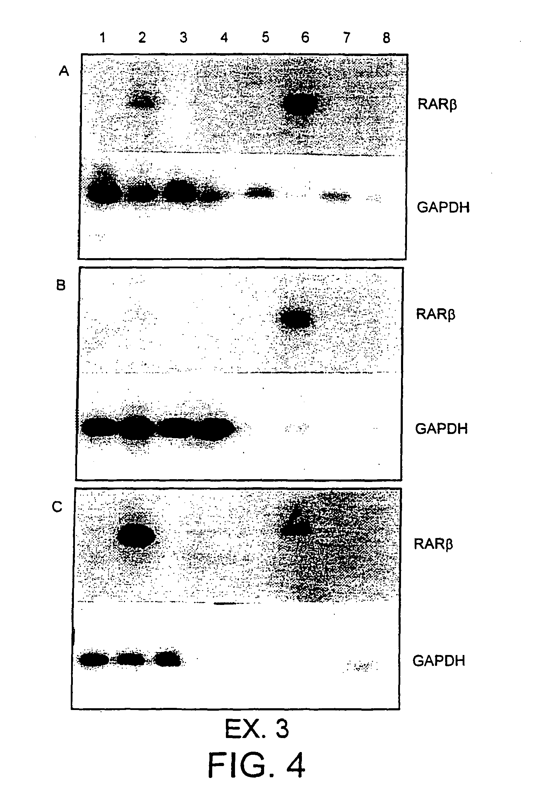

Three different transfections were performed, two of which served as controls. Firstly, just the vector containing lacZ (pHSVlacZ); secondly the vector containing RARβ2 (pHSVRARβ2) ; thirdly the vector containing another isoform of the RARb gene, RARβ4 (pHSVRARβ4). The latter served as a very precise control for transfection since we do not detect the RARβ4 isoform after RA treatment of neurons in our previous experiments²⁰ hence it is not involved in neurite outgrowth. We first ensured that the transfections were successful and that the relevant receptor isoform was expressed in the cultured cord. Pieces of spinal cord were transfected overnight with the appropriate construct and analysed either three or four days later. The pHSVlacZ treated cords showed a significant amount of transfection had taken place as judged by b-galactosidase staining of the adult cord (Fig. 3B). RT-PCR demonstrated that transfection with the RARβ2 vector resulted in the expression of RARβ2 (Fig. 4, lane 3) but not RARβ4 (Fig. 4, lane 4) and transfection with the RARβ4 vector resulted in the expression of RARβ4 (Fig. 4, lane 8) but not RARβ2 (Fig. 4, lane 7). In the non transfected cord neither RARβ2 or RARβ4 were detected (Fig. 4, lanes 2 and 6).

The effects of these transfections on neurite outgrowth were clear-cut. Transfection with the pHSVlacZ failed to change the behaviour of the cultured adult cord which remained completely un-responsive in terms of neurite outgrowth (Fig. 5A, 12/12 transfections). Similarly, the transfections with pHSVRARβ4 produced no response in the cultured cord which remained inert (Fig. 5C, 12/12 transfections). However, transfections with the pHSVRARβ2 isoform clearly produced a different behaviour and many neurites appeared in the cultures (Fig. 5B, 8/12 transfections). The number of neurites produced in the pHSVRARβ2 cord varivaried between 3 and 23. In the pHSVIacZ transfections there was considerable variability in the number of lacZ-positive cells per explant. This suggests that the variability in neurite number may be due to variability in number of cells transfected.

These results provide strong support for our hypothesis that the RARβ2 isoform plays a key role in the induction of neurite outgrowth in response to RA and that this may be a crucial component which fails to be up-regulated in the injured adult CNS. Our hypothesis is based upon several experiments involving either regenerating or non-regenerating neuronal tissues and their response to RA. Thus the embryonic mouse spinal cord, the embryonic mouse DRG and the adult mouse DRG all respond to RA by up-regulating RARβ2 and extending neurites. In contrast, the adult mouse spinal cord fails to up-regulate RARβ2 and fails to extend neurites. Furthermore, NGF stimulates neurite outgrowth and acts by up-regulating RARβ2 ²⁴ and neurite outgrowth from embryonic mouse DRG can be stimulated by a RARβ agonist²⁰.

These results reveal that when the genome of the neuron itself is manipulated then regeneration can be reawakened. This is in contrast to the recent inductions of neurite outgrowth *in vivo* which have concentrated on the inhibitory factors present in the CNS environment¹⁻⁵. During development the loss of regenerative capacity of the spinal cord correlates with the appearance of myelin associated neurite growth inhibitory molecules and some of these are thought to be produced by the oligodendrocytes²⁵. Either the regeneration of neurites we see in our cultures and that seen in cultures where the environment has been manipulated are two different mechanism of neurite regeneration or they are related processes. Support for the latter view is provided by the fact that CNS neurons themselves have been shown to be involved in myelination²⁶. Therefore it is tempting to speculate that the presence of RARβ2 in neurons during development may regulate genes involved in myelination, and that this process is recapitulated by transfection of the RARβ2 gene in the adult CNS.

None of the neurites we observed in the RARβ2 transfected cord elongated over an appreciable distance. This suggests that elongation of the neurite may require the expression of a different set of genes. Evidence that this may be true is shown from regeneration of axons in the adult PNS, where a transition from arborizing to elongating growth depends upon a transcriptional dependent switch²⁷. Alternatively the failure of elongation of neurites in our cultures may be due to the fact that there is likely to be a loss of expression of RARβ2 over time due to the transient nature of the transfections and that this does not allow enough time for elongation to occur.

Nonetheless we propose that these preliminary data support a role of RARβ2 in the regeneration of neurites in the adult CNS and that gene therapy with this transcript in combination with other treatments may one day lead to functional recovery of the injured spinal cord.

### Methods

**Cultures.** Spinal cord was dissected from either E13.5 or 10 month old mice and cut into transverse pieces of about 5 mm. These were cultured in cellogen matrix (ICN flow), prepared by mixing 1 volume of 7.5 % sodium bicarbonate, 1 volume of 10x MEM (Gibco) and 8 parts cellogen (ICN flow). The pH was adjusted to 7.5 by dropwise addition of 5M NaOH. Explants were fed every two days. The media consisted of DMEM-F12 with glutamine (Gibco), 6 % glucose, GMS-A (Gibco) 10% delipidated serum and *all-trans-*RA (stock solution, 1 x 10⁻⁵ M, Sigma). On the fifth day they were fixed in 4 % paraformaldyde and stained with the neurofilament antibody, NF200 (Sigma).

**RT-PCR analysis.** RNA was extracted (trizol, Gibco) and cDNA prepared by the use of a Pharmacia kit as described in the manufactures instructions. The primers used were from GAPDH, RARβ2 and RARβ4, (details upon request). PCR was carried out for 25 cycles for embryonic spinal cord and 40 cycles for adult spinal cord. Amplification was carried out as follows, denaturation for 30 s at 95 °C, annealing for 30 s at 55 °C and extension for 1 min at 72 °C. One fifth of the resultant product was then run on a gel.

**Transfections.** Virus stocks were prepared and B galactosidase staining carried out as described in ref²⁸. The titres used were: pHSV RARβ2, 5x10⁻⁴ ip/ul, pHSVRARβ4" 4x10⁻⁴ ip/ ul, pHSVlacz 5x10⁻⁴ ip/ul.

### FIGURES FOR EXAMPLE 2:

Fig.1. Comparison of the effect of retinoic acid on neurite outgrowth on cultured E13.5 (A, C, E, G) and 10 month old adult spinal cord (B, D, F, H). Pieces of spinal cord were cultured in cellogen in the presence of 10 % delipidated serum and RA for a period of five days. The medium was changed every two days. **A, B,** no RA; **C, D**, 1 x 10⁻⁸ M RA: **E, F**, 1 x 10⁻⁷ M RA; **G, H**, 1 x 10⁻⁶ M RA.
Fig.2. Expression of RARβ2 in E13.5 and 10 month old adult spinal cord. Pieces of spinal cord were cultured in the presence of various concentrations of RA for a period of five days after which time RT-PCR analysis of RARβ2 was performed. A. E13.5 (lanes 2-5) and B. 10 month old adult spinal cord (lanes 2-5). Lanes: 1. bluescript/HPA II size markers, 2. no RA, 3. 1 x 10⁻⁸ M RA, 4. 1 x 10⁻⁷ M RA, 5. 1 x 10⁻⁶ M RA. The presence of GAPDH was used to indicate equal amounts of cDNA in the samples.
Fig.3. Transfection of adult spinal cord with pHSVlacZ. Cultured 10 month old adult spinal cord was transfected with x 10⁻⁴ ipu/ul pHSVlacZ overnight and analysed for B galactosidase staining 3 days later. A. non-transfected adult spinal cord. B. adult spinal transfected with pHSVlacZ.
Fig.4. Transfection of adult spinal cord with either pHSVRARβ2 or pHSVRARβ4. Adult spinal cord was cultured in cellogen and transfected either with 5 x 10⁻⁴ ipu/ul of pHSVRARβ2 or 4 x 10⁻⁴ ipu/ul pHSVRARβ4 overnight. RT-PCR analysis four days after transfection, of RARβ2 (lanes 2-4) and RARβ4 (lanes 6-8) expression in adult spinal cord transfected with Lanes 2, 6 no virus . 3, 7 pHSVRARβ2, 4, 8, pHSVRARβ4. The presence of GAPDH was used to indicate equal amounts of cDNA in the samples. Lanes 1,2 bluescript/HPA II size markers.
Fig.5. Effect of either pHSVIacZ, pHSVRARβ2 or pHSVRARβ4 transfection in cultured adult spinal cord on neurite outgrowth. Ten month old spinal cord was cultured in cellogen and transfected with either 5 x 10⁻⁴ ipu/ul, pHSVlacZ, x 10⁻⁴ ipu/ul, pHSVRARβ2 or 4 x 10⁻⁴ ipu/ul pHSVRARβ4 overnight, and analysed for neurite staining with NF200 4 days after transfection. Cultured spinal cord transfected with A. pHSVlacZ, B. pHSVRARβ2, C. pHSVRARβ4.
Fig. 6 is a barchart of the average number of neurites per spinal cord explant.

### EXAMPLE 3: NEURITE OUTGROWTH FROM MOUSE GANGLIAL NEURONES

The role of retinoic acid receptors in neurite outgrowth from different populations of embryonic mouse dorsal root ganglia.

Dorsal root ganglion (DRG) neurons can be categorised into at least three types based upon their neurotrophin requirement for survival. We have analysed the expression of the retinoic acid receptors (RARs) and the retinoid X receptors (RXRs) in NGF, NT-3 and BDNF dependent neurons isolated from embryonic day 13.5 mouse DRG. We show that each population of neurons expressed each of the three RXRs, α, β and γ. However, whilst the NGF and NT-3 dependent neurons expressed each of the RARs α, β and γ the BDNF dependent neurons only expressed RARα and β. When retinoic acid was added to each of the neuronal classes only the NGF and NT-3 dependent neurons responded by extending neurites, and this response involved the up-regulation of RARβ2. This specificity was confirmed by the use of receptor selective agonists as only a RARβ selective compound stimulated neurite outgrowth. These results suggest a role for RA acting via RARβ2 in the outgrowth of neurites.

### Introduction

The neurotrophins are a family of growth factors that are required for the survival of a variety of primary sensory neurons in the developing peripheral nervous system (Snider, 1994). The family includes nerve growth factor (NGF) (Levi-Montalcini, 1987) neurotrophin-3 (NT-3) (Maisonpierre et al., 1990) and brain-derived neurotrophic factor (BDNF) (Barde et al., 1982). They are synthesised in the target fields innervated by peripheral neurons and are thought to be transported by a retrograde mechanism from the target field to support the survival of the developing neurons. The neurotrophins act through receptor tyrosine kinases designated Trk. NGF specifically activates TrkA; BDNF activates TrkB and NT-3 activates TrkC (reviewed in Snider, 1994). Analysis of the phenotypes resulting from loss of function experiments of the neurotrophins and the receptor tyrosine kinases have revealed that the dorsal root ganglia (DRG) neurons can be classified into at least three types. Neurons that require NGF for their survival mediate nocioceptive (pain) and thermal receptive functions. In the periphery the axons terminate in the superficial layers of the skin and innervate the superficial laminae of the spinal cord (Crowley et al., 1994; Smeyne et al., 1994). Proprioceptive neurons (sense of position of the limbs in space), which are much larger than NGF type neurons, project into the periphery to the primary endings of muscle spindles and extend a collateral branch to the motor pools in the spinal cord are dependent upon NT-3 for their survival (Ernfors et al., 1994; Farinas et al., 1994; Klein et al., 1994). BDNF neurons are small to medium sized and may include some classes of the mechanoreceptors (Klein et al., 1993; Jones et al., 1994).

In addition to growth factors being involved in the survival of neurons, retinoids can also carry out the same role. Retinoids are a family of molecules derived from vitamin A and include the biologically active metabolite, retinoic acid (RA). The cellular effects of RA are mediated through the action of two classes of receptors, the retinoic acid receptors (RARs) which are activated both by all*-trans-*RA (*t*RA) and 9-*cis*-RA (9-*cis*-RA), and the retinoid X receptors (RXRs), which are activated only by 9-*cis-*RA (Kastner et al., 1994; Kleiwer et al., 1994). The receptors are of three major subtypes, α, β and γ, of which there are multiple isoforms due to alternative splicing and differential promoter usage (Leid et al. 1992). The RARs mediate gene expression by forming heterodimers with the RXRs, whilst the RXRs can mediate gene expression as homodimers or by forming heterodimers with a variety of orphan receptors (Mangelsdorf & Evans, 1995). Interestingly, one of the earliest genes induced by NGF in PC12 cells is the orphan receptor NGFI-B (NURR1) (Millbrandt, 1989). This suggests that the growth factor and retinoid mediated pathway in developing neurons can interact. This interaction may be critical for the survival of the neuron because RA has been shown to be involved in the survival and differentiation of neurons (Wuarin and Sidell, 1991; Quinn and De Boni, 1991). Furthermore, many studies on a variety of embryonic neuronal types have shown that RA can stimulate both neurite number and length (reviewed in Maden, 1998) as indeed, can the neurotrophins (Campenot, 1977; Lindsay, 1988; Tuttle and Mathew, 1995). Recently we have shown that RA is critical for neurite regeneration in adult DRG and that it's synthesis may be regulated by NGF (Corcoran and Maden, 1999).

In the work described here, we use E13.5 mouse DRG to investigate further the nature of the interaction between the retinoid mediated pathway and the growth factor pathway by asking which of the RARs and RXRs are expressed in neurons that are dependent upon different neurotrophins for their survival. We show that a different retinoid receptor profile is indeed expressed in NGF, NT-3 and BDNF neurons and that this profile is altered after an RA treatment which induces neurite outgrowth. Specifically, RARβ2 is up-regulated in NGF and NT-3 neurons but not in BDNF type neurons. This result was confirmed by the use of receptor selective agonists, as only the RARβ agonist will substitute for RA in inducing neurite outgrowth. These results suggest a role for RA acting via RARβ2 in the outgrowth of neurites.

### Materials and Methods

**DRG cultures.** DRG were obtained from E13.5 mice, freed of non-ganglionic tissue and collected in ice-cold calcium magnesium free phosphate buffered saline. To prepare dissociated cell suspensions the ganglia were treated with 0.05 % trypsin for 15 minutes at 15 °C. The reaction was stopped by the addition of 1% serum and single cells obtained by trituration with a 23 G needle. The cells were then spun at 1000 g for ten minutes and resuspended in media. They were plated out at a density of approximately of 25000 cells/cm² in wells that had been precoated with 100µg/ml poly D lysine for 2 hrs. The cultures were fed every 2 days.

Culture media consisted of DMEM-F12 with glutamine (Gibco), 6 % glucose, ITS (Gibco). The growth factors used were either 50ng/ml NGF (7s, Promega) 50ng/ml NT3 (Promega) or 50ng/ml BDNF (Promega). Retinoids were used at a concentration of 1 x 10⁻⁷ M. All-*trans*-retinoic acid was obtained from Sigma and the receptor agonists were synthesised by CIRD Galderma: CD366 activates RARα, CD2019 activates RARβ, CD437 activates RARγ and CD2809 activates all of the RXRs.

**RT-PCR analysis.** RNA was extracted (trizol, Gibco) and cDNA prepared by the use of a Pharmacia kit as described in the manufacturer's instructions. The primers used were from mouse RARs, RXRs and GAPDH (details upon request). In order to identify which RAR/RXR receptors were involved neurite outgrowth semi quantitative PCR was used. Amplification was carried out in the linear range for each RAR and RXR and their levels of expression were compared to GAPDH. For the RXRs 28 cycles were performed, while 25 cycles were used for RARα and RARγ and 22 cycles for RARβ and GAPDH. Amplification was carried out as follows, denaturation for 30 s at 95 °C, annealing for 30 s at 55°C and extension for 1 min at 72 °C. One fifth of the resultant product was then run on a gel and blotted. This was then probed with the appropriate RAR, RXR or GAPDH for normalisation.

**In situ Hybridisation:** Cells were washed once with PBS and fixed in 4% PFA for 30 mins. They were then washed twice for 5 mins in PBS-0.05% Tween (PBT). Hybridisation was carried out at 55°C overnight. The buffer consisted of 0.1M Tris-Cl, pH9.5, 0.05M MgCl₂, 0.1M NaCl and 0.1% Tween-20. The cells were then washed sequentially for 15 min. at 65°C in 50 % hybridisation buffer, 50% 2x SSC, 100% 2x SSC, and finally in 0.2% SSC. They were then washed at RT for 5 minutes each in 75% 0.2x SSC, 25% PBT, 50% 0.2x SSC, 50% PBT, 25% 0.2x SSC, 75% PBT, and 100% PBT. The cells were blocked in 2% sheep serum in PBT for 1 hr and incubated with anti DIG antibody overnight at 4°C. The cells were then washed 8 times in PBT for 2 hrs. Colour was developed by using NBT/BCIP according to the manufacturer's instructions (Boehringer Mannheim).

**Immunohistochemistry and measurement of neurite length:** Cells were washed once with PBS and fixed in 4% PFA for 30 mins. They were then washed twice for five minutes in PBS-0.05% Tween (PBT). They were then incubated in primary antibody NF200 (sigma) at 4°C overnight and washed 8 times for 2 hrs in PBT. Secondary antibody was then applied for 2 hrs at RT, and the cells again washed 8 times for 2 hrs in PBT. They were then incubated for 5 mins. in PBS containing 0.5 mg/ml DAB and 6% H₂O₂. Neurite length was measured by using NIH image software. The experiments were repeated three times and three random fields were taken for each experiment for analysis. On average there were 40 neurons in each field and the longest neurite branch was measured for a given neuron.

### Results

### Expression of receptors by in situ hybridisation.

We first examined the expression of the RARs and the RXRs in primary cultures of E13.5 mouse DRG by in situ hybridisation. Dissociated DRG neurons were cultured in serum free medium either in the presence of NGF, NT-3 or BDNF for a period of five days. In the absence of neurotrophins the cells died. We found that all three types of neurons expressed RXRα (Fig. 1 D, J, P), RXRβ (Fig. 1 E, K, Q) and RXRγ (Fig. 1 F, L, R). In contrast, the RARs showed a differential expression between the three types of neurons. Whilst the NGF and NT-3 dependent neurons expressed RARα (Fig. 1 A, G), RARβ (Fig. 1 B, H) and RARγ (Fig. 1 C, I), the BDNF dependent neurons only expressed RARα (Fig. 1 M) and RARβ (Fig. 1 N). RARγ was not detectable by in situ hybridisation in the BDNF dependent cultures (Fig. 1 O).

### Effect of RA on neurite outgrowth

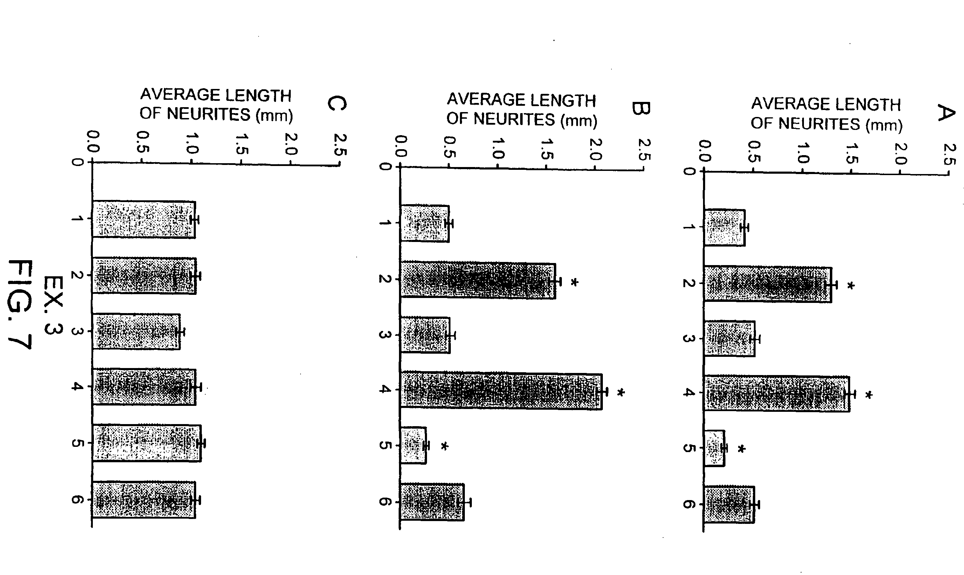

In order to eliminate any trophic effect of RA on the different populations of neurons we grew the neurons in serum free medium plus the relevant neurotrophin for a period of two days before adding 1 x 10-⁷M RA to the cultures for 3 days. Control cultures had no RA added and were maintained in neurotrophin only. There was no significant difference in the numbers of neurons cultured in the presence or absence of RA. This suggests that the effect of RA was on neurite outgrowth and not due to the selective survival of subsets of neurons under the different culture conditions used. In order to analyse neurite outgrowth the cultures were fixed after five days and stained with the monoclonal antibody NF200. Neurite length was measured by NIH image software. The experiment was repeated three times. In total approximately 120 neurons were counted in each experiment and the longest neurite was measured from each neuron from which an average neurite length was taken for each treatment. In the absence of RA the BDNF dependent neurons (Fig. 2 E and Fig.7 C column 1) grew neurites whilst the NGF (Fig. 2 A) and NT-3 dependent neurons (Fig. 2 C) showed limited neurite outgrowth (Fig. 7 A and B column 1). In contrast, when RA was added to the medium there was a dramatic increase in the length and number of neurites in the NGF (Fig. 2 B) and NT-3 dependent neurons (Fig. 2 D) and this difference was found to be significant when the length of the neurites were compared (Fig 7 A and B, columns 1 and 2). In contrast RA had no affect on neurite outgrowth of the BDNF dependent neurons (Fig. 2 F and fig. 7 C columns 1 and 2).

### Expression of receptors and response to RA by RT-PCR

In order to identify which of the receptors are involved in neurite outgrowth semiquantitative PCR was carried using primers against the RXRs and the individual RAR isoforms as described in the materials and methods. There was no difference in the expression of the RXRs in each of the three types of neurons cultured with or without RA. In contrast, there were variations in the RAR receptor profiles. Each of the three types of neurons expressed RARα₁ (Fig. 3 A, B, C, lane 1) which was strongly up-regulated in response to RA in the NGF (Fig. 3 A, lane 8) and NT-3 (Fig. 3 B, lane 8) dependent neurons and only slightly up-regulated in the BDNF dependent neurons (Fig. 3 C, lane 8). It is clear from Fig. 3 that only the RARα₁ isoform is readily detectable in these DRG neurons although on over-exposure of the blots the NT-3 dependent neurons expressed the RARα₅ and RARα₇ isoforms and the BDNF dependent neurons expressed the RARα₆ and RARα₇ isoforms.

Of the four possible RARβ isoforms only the RARβ₂ isoform was detected in all three types of neuron. This isoform was strongly up-regulated by RA in the NGF (Fig. 4 A, lane 6) and NT-3 dependent neurons (Fig. 4 B, lane 6) but not in the BDNF dependent neurons (Fig. 4 C, lane 6) as compared to the non-stimulated cultures (Fig. 4 A, B, C, lane 2)..

Of the seven RARγ isoforms only RARγ₁ isoform was detected in the neuronal cultures and then only in the NGF (Fig. 5 A, lane 1 and 8) and NT-3 dependent neurons (Fig. 5 B, lane 1 and 8). No RARγ₁ was detected by RT-PCR in the BDNF dependent neurons.

### Receptor selective analogues and neurite outgrowth

The above data suggested that the up-regulation of either RARα₁ or RARβ₂ may be responsible for the increase of neurite outgrowth observed in the NGF and NT-3 dependent neurons (Fig. 2 B, D). It is more likely to be the RARβ₂ isoform since this receptor is not upregulated in the BDNF dependent neurons and there is no increase in neurite outgrowth when these are stimulated with RA (Fig. 2 F) whereas the RARα1 isoform is up-regulated despite a lack of neurite response to RA. In order to distinguish between these two receptors we used receptor selective synthetic retinoids which have been developed specifically to activate individual receptors. CD366 activates RARα, CD2019 activates RARβ, CD437 activates RARγ and CD2809 activates all of the RXRs.

In the presence of the RARα agonist there was no significant increase in neurite outgrowth in any neuronal population (Fig. 6 A, E, I and Fig 7 A B and C columns 1 and 3). In contrast, the RARβ agonist significantly increased neurite outgrowth in the NGF and NT-3 dependent neurons compared to non treated neurons (Fig. 6 B, F and Fig 7 A B columns 1 and 4), but did not effect neurite outgrowth in the BDNF dependent neurons (Fig. 6 J and Fig. 7 C columns 1 and 4). When the different neuronal populations were cultured in the presence of the RARγ agonist there was significant decrease in neurite outgrowth in the NGF and NT-3 dependent neurons (Fig. 6 C, G and Fig. 7 A B columns 1 and 5) whereas neurite outgrowth still occurred in the BDNF dependent neurons (Fig. 6 K and Fig. 7 C columns 1 and 5). There was no significant effect on neurite outgrowth in any of the neuronal populations when they were cultured in the presence of the RXR agonist (Fig. 6 D, H, L and Fig. 7 A B and C columns 1 and 6).

Therefore, in agreement with our RT-PCR data RARβ₂ is required for neurite outgrowth. Furthermore, RARγ can inhibit neurite outgrowth.

### Interrelationships between RARs

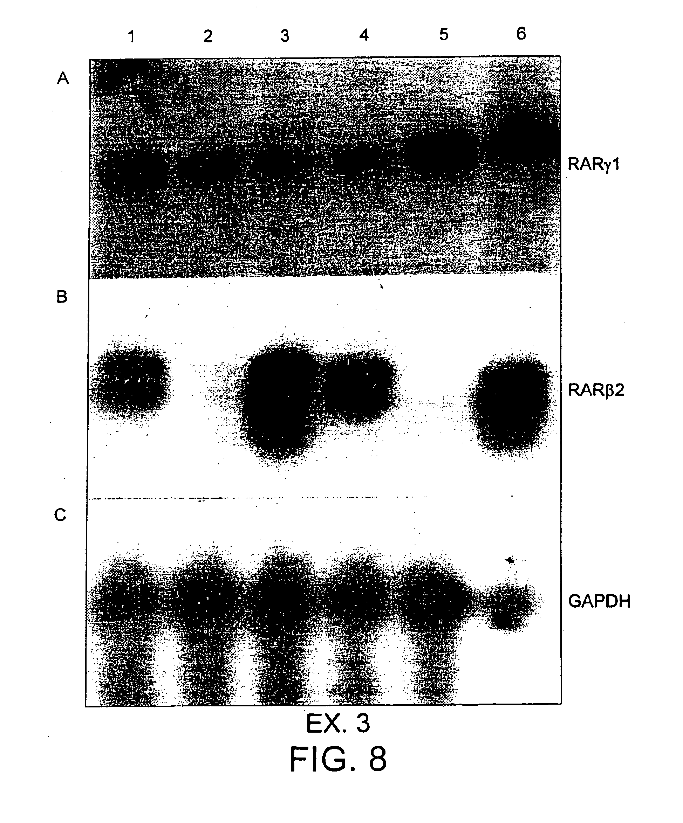

Finally, we attempted to investigate whether there were any regulative interactions between the receptors RARβ2 and RARγ1 since these have opposite affects on neurite outgrowth. In order to examine this we cultured NGF and NT-3 dependent neurons in serum free medium in the presence of either the RARγ agonist or the RARβ agonist and looked at the levels of receptor expression by semi quantitative RT-PCR 24 hrs. later. The RARβ agonist up-regulated the expression of RARβ2 in both the NGF and NT-3 dependent neurons (Fig. 8 B, lanes 3 and 6) compared to non-stimulated cultures (Fig.8 lanes 1 and 4) but did not affect the expression of RARγ₁ (Fig. 7 A, lanes 3 and 6). However, in the presence of the RARγ agonist, RARβ₂ is reduced in both the NGF and NT-3 dependent neurons (Fig. 8 B, lanes 2 and 5) compared to non-stimulated cultures (Fig. 8 B, lanes 1 and 4). The RARγ agonist had no effect on the level of RARγ₁ (Fig. 8 A, lanes 2 and 5). Thus RARγ1 can regulate the expression of RARβ2.

### Discussion of Example 3

Our results show that each of the three dorsal root ganglia neuronal populations we have isolated (NGF, NT-3 and BDNF dependent) express both a common set and a unique set of retinoid receptors. With regard to the RXRs they each express RXRα, RXRβ and RXRγ and none of these were found to be directly involved in neurite outgrowth. In contrast, the neurons expressed different RARs depending on the neurotrophin used to select them. The major RAR isoforms that were common to each population were RARα₁ and RARβ₂. In addition, the NGF and NT-3 populations expressed RARγ₁ which was not expressed in the BDNF population at the time point analysed.

Only the NGF and NT-3 dependent neurons responded to RA by extending neurites whereas the BDNF dependent neurons produced neurites irrespective of the presence or absence of RA. In parallel there was a change in the RAR profile after RA addition. RARα₁ and RARβ₂ were strongly up-regulated in the NGF and NT-3 dependent neurons whereas in the BDNF dependent neurons only the RARα₁ was upregulated. This suggested that RARβ₂ was required for the induction of neurite outgrowth and to confirm this observation we used receptor selective agonists.

The development of receptor selective agonists has provided an extremely valuable tool to begin to examine the role of individual receptors in any particular biological process. We showed here that only the RARβ agonist, CD2019, mimiced the effect of RA by inducing neurite outgrowth in NGF and NT-3 neurons thus confirming our RT-PCR results.

We also observed that the RARγ agonist caused a decrease in neurite outgrowth of the NGF and NT-3 dependent neurons. In an attempt to show whether this was associated with the RARβ₂ expression we examined whether the RAR agonists had any effect on receptor expression. The RARβ agonist upregulated the expression of RARβ₂ but had no effect on the expression of RARγ1. In contrast whilst the RARγ agonist had no effect on the expression of RARγ1 it did down-regulate the level of RARβ2 expression, this phenomenon may also be a prelude to neurite outgrowth. This suggests that the RARβ transcript can be regulated by RARγ/RXR heterodimers. The lack of increase in neurite outgrowth in response to RA of the BDNF dependent neurons also suggests that in this type of neuron that RARβ may be regulated differently to RARβ in the NGF and the NT-3 dependent neurons at the embryonic stage studied.

Our results suggest that it is the activation of the RAR pathway that is responsible for neurite outgrowth, since a RXR agonist which activates RXR/orphan receptors had no effect on neurite outgrowth whereas the RARβ agonist which activates RAR/RXR heterodimers increased the amount of neurite outgrowth. This suggest that if NGF acts via the RXR/orphan receptor pathway by utilising for example NGFI-B then it is not, in these embryonic stages, directly responsible for neurite outgrowth, rather it may be required for neuron survival. Interestingly in the adult the contrast seems to be true. NGF is not required for neuron survival but it is required for neurite outgrowth (Lindsay, 1988). Thus there may be different mechanisms for neurite outgrowth in developing and adult regenerating neurites. However, there is a absolute requirement for RA in neurite outgrowth during development. In the vitamin A deficient quail the neural tube fails to extend neurites into the periphery (Maden et al. 1996; Maden et al., 1998).

The differential response of these neurons to RA and the receptor agonists may have some significance for embryonic and adult tissues which require retinoids for their development and/or survival. In order to activate different RAR/RXR and RXR/orphan receptor combinations there may be different retinoids present in the tissues. Some support for this view is provided by the fact that there are numerous RA generating enzymes which show localised expression during development (McCaffery et al., 1992; Drager & McCaffery, 1995; Godbout et al., 1996; Neiderreither et al., 1997; Ang & Duester, 1997) and each of these enzymes could make different retinoids. Several novel retinoids have so far been discovered, for example 5, 6-epoxyretinoic acid, which is found in the intestine (McCormick et al., 1978), 4-*oxo-*retinol which is the biologically active metabolite that is responsible for the differentiation of murine embryonic F9 cells (Achkar et al., 1996) and 14- hydroxy-4, 14-retroretinol which is found in B lymphocytes (Buck et al., 1991).

Therefore, the embryo may be able to regulate the amount of neurite outgrowth by synthesising different retinoids. By activating RARβ₂/RXR heterodimers neurite outgrowth could occur whereas by activating RARγ/RXR heterodimers neurite outgrowth could be stopped. In addition the amount of neurite outgrowth could be regulated by the amount of retinoic acid. For example in the developing mouse spinal cord there are high concentrations of retinoids in the brachial and lumbar enlargements (McCaffery & Drager, 1994). This may be an intrinsic requirement for innervation of the extremities of the limb where the neurites have to travel large distances to reach their final targets, whereas in the thoracic region where the concentration of retinoids are lower such extensive neurite outgrowth would not be required.

### Figure Legends for Example 3

Figure 1. Expression of the RARs and RXRs by E13.5 mouse embryo DRG neurons cultured either in the presence of NGF, NT-3 or BDNF. In situ hybridisation of: **A-F,** NGF neurons; **G-L,** NT-3 neurons; **M-R,** BDNF neurons. Expression of : **A, G, M,** RARα; **B, H, N,** RARβ; **C, I, O**, RARγ; **D, J, P**. RXRα; **E, K,** , RXRβ; **F, L, R,** RXRγ.
Figure 2. Effect of RA on neurite outgrowth from DRG neurons. DRG neurons were cultured either in the presence of NGF, NT-3 or BDNF for a period of two days at which point 1 x 10⁻⁷ M all*-trans-*RA was added. They were then examined for neurite outgrowth after a total of five days with NF200 antibody. **A,** NGF; **B,** NGF + 1 X 10-7 M RA; C, NT-3; **D,** NT-3 + 1 X 10⁻⁷; **E,** BDNF; **F,** BDNF + 1 X 10⁻⁷ M RA.
Figure 3. Expression of RARα isoforms in DRG neurons cultured either in the absence or presence of RA. DRG neurons were cultured in the presence of either NGF, NT-3 or BDNF for a period of two days, 1 X 10⁻⁷ M RA was then added and the presence of the RARα isoforms were then assayed by RT-PCR. Controls had no RA added. A, control NGF neurons lanes 1-7; NGF neurons + 1 X 10⁻⁷ M RA lanes 8-14. **B**, control NT-3 neurons lanes 1-7; NT-3 neurons + 1 X 10⁻⁷ M RA lanes 8-14. **C**, control BDNF neurons lanes 1-7; BDNF neurons + 1 X 10⁻⁷ M RA lanes 8-14. Lanes: 1 & 8, RARα1; 2 & 9, RARα2; 3 & 10, RARα3 ; 4 & 11, RARα4; 5 & 12, RARα5; 6 & 13, RARα6; 7 & 14, RARα7.
Figure 4. Expression of RARβ isoforms in DRG neurons cultured either in the absence or presence of RA. DRG neurons were cultured in the presence of either NGF, NT-3 or BDNF for a period of two days, 1 X 10⁻⁷ M RA was then added and the presence of the RARβ isoforms were then assayed by RT-PCR. Controls had no RA added. **A,** control NGF neurons lanes 1-4; NGF neurons + 1 X 10⁻⁷ M RA lanes 5-8. **B,** control NT-3 neurons lanes 1-4; NT-3 neurons + 1 X 10⁻⁷ M RA lanes 5-8. **C,** control BDNF neurons lanes 1-4; BDNF neurons + 1 X 10⁻⁷ M RA lanes 5-8. Lanes: 1 & 5, RARβ1; 2 & 6, RARβ2; 3 & 7, RARβ3; 4 & 8, RARβ4.
Figure 5. Expression of RARγ isoforms in DRG neurons cultured either in the absence or presence of RA. DRG neurons were cultured in the presence of either NGF, NT-3 or BDNF for a period of two days, 1 X 10⁻⁷ M RA was then added and the presence of the RARγ isoforms were then assayed by RT-PCR. Controls had no RA added. **A,** control NGF neurons lanes 1-7; NGF neurons + 1 X 10⁻⁷ M RA lanes 8-14. **B,** control NT-3 neurons lanes 1-7; NT-3 neurons + 1 X 10⁻⁷ M RA lanes 8-14. Lanes: 1 & 8, RARγ1; 2 & 9, RARγ2; 3 & 10, RARγ3; 4 & 11. RARγ4; 5 & 12, RARγ5; 6 & 13, RARγ6; 7 & 14, RARγ7.
Figure 6. Effect of RAR and RXR agonists on neurite outgrowth from DRG neurons. DRG neurons were cultured either in the presence of NGF , NT-3 or BDNF for a period of two days at which point either 1 x 10⁻⁷M of either CD366 (RARα agonist), CD2019 (RARβ agonist), CD437 (RARγ agonist) or CD2809 (pan-RXR agonist) were added to the cultures. Cultures were then stained for neurite outgrowth at five days with the NF200 antibody. **A-D,** NGF type neurons; **E-H,** NT-3 type neurons; **I-L,** BDNF type neurons. Agonists: RARα **A, E, I;** RARβ **B, F, J;** RARγ **C, G, K;** RXR **D, H, L.**
Figure 7. Effect of retinoid agonists on the length of neurites from **A.** NGF type neurons; **B.** NT-3 type neurons, **C.** BDNF type neurons. Columns 1. no agonist, 2. RA, 3. RARα, 4. RARβ, 5. RARγ, 6. RXR. Error bars s.e.m., n = 50. *p < 0.01.
Figure 8. Effect of a RARγ or RARβ agonist on the expression of RARγ1 and RARβ2 expression in DRG neurons cultured in the presence of NGF or NT-3. DRG neurons were cultured in the presence of serum free medium. After two days 1 x 10⁻⁷M RARγ or RARβ agonist were then added to the cultures for a period of 24 hrs. RT-PCR analysis of **A.** RARγ1; **B,** RARβ2 expression in NGF (lanes, 1-3) and NT-3 (lanes, 4-6) type neurons. Lanes: 1,4, no agonist; 2,5, RARγ agonist; 3, 6. RARβ agonist

### SUMMARY

The Examples demonstrate that RARβ2 can be used to cause neurite development

When peripheral nerves are damaged some regeneration can occur unlike nerves of the central nervous system which show no regeneration. However regeneration of peripheral nerves is limited particularly when there is traumatic nerve injury where there is a loss of nerve tissue such that a gap is created which the regenerating neurite cannot grow across. This delay in nerve regeneration can lead to muscle atrophy and lead to permanent disability.

In response to peripheral nerve injury neurotrophins are produced. These are a family of growth factors that are required for the survival of a variety of neurons. The family includes nerve growth factor (NGF) neurotrophin-3 (NT-3) and brain-derived neurotrophic factor (BDNF). It was hoped that neurotrophins could be used in the treatment of PNS injuries. However the results have not been encouraging. Two major problems have been encountered, firstly the problem of delivery to the injury, and secondly since different neurons need different neurotrophins a cocktail of them as to be administered in order for all the nerves to regenerate. We have investigated how neurotrophins stimulate neurite regeneration.

We have found that the vitamin A derivative all-trans-retinoic acid (tRA) like NGF induces neurite outgrowth from various embryonic sources, including PNS. Cellular effects of the tRA are mediated by binding to nuclear receptors that are ligand activated transcription factors. There are two classes of receptors, retinoic acid receptors (RARs) and retinoid X receptors (RXRs), with three subtypes of each: α, β and γ. RAR receptors mediate gene expression by forming heterodimers with RXRs, whereas RXRs can mediate gene expression either as hormodimers or by forming heterodimers with orphan receptors.

We have found that only RARβ2 is required for neurite outgrowth of all types of neurons we have cultured. Furthermore when adult mouse DRG are cultured in the presence of NGF and an inhibitor of tRA synthesis, neurite outgrowth does not occur. Conversely, when tRA is added along with a blocking antibody to NGF, neurite outgrowth occurs as normal. We have also shown that NGF induces transcription of both the tRA-synthesizing enzyme RALDH-2 and the RARβ2 as well as a detectable release of sythesized tRA.

We propose that the stimulation of RARβ2 is an intrinsic requirement for the regeneration of neurites in the peripheral nervous system and that crucially this is downstream of the neurotrophins.

We have extended our observations to the CNS. We have found that the embryonic spinal cord expresses RARβ2 and that the amount of its expression correlates with the amount of neurite outgrowth. In contrast the adult spinal cord does not express RARβ2 nor an it regenerate neurites. We have shown that by transfecting RARβ2 by use of a defective herpes simplex virus type 1 (HSV-1) vector into cultured adult spinal cord we have transformed the normally inert spinal cord into one which can extend neurites. Therefore, we propose that gene therapy of injured spinal cord with RARβ2 will lead to functional recovery.

Gene therapy with RARβ2 to treat CNS injuries should lead to functional recovery and therefore the prevention of paralysis.

PNS and CNS injuries occur all over the world unfortunately it is unlikely that the incidence of such injuries will decrease. World wide a 1000 people per million of the population a year suffer spinal cord injury, ten times this number suffer some sort of PNS injury.

In addition there are three other areas where RARβ2 would be of use. In leprosy, diabetes and AIDS, neuropathy occurs (the neurites die) this is equivalent to PNS injury. In both leprosy and diabetes it has been shown that there is a loss of NGF in the skin of both types of patients leading to the loss of pain and sensation and inflammation which can lead to ulcer formation. In AIDS patients sensory neuropathy is one of the most common effects of the HIV infection, already NGF as been used to treat this condition.

In summation, our results indicate a role for RA acting via RARβ2 in the outgrowth of neurites from certain classes of neurons.

The present invention comprises the use of RARβ2 in the preparation of a medicament for the treatment of neurodegenerative disease. In the treatment, expression of the retinoic acid receptor RARβ2 is ensured in affected cells or tissues. This may be achieved by gene therapy i.e. insertion of nucleic acid coding for this receptor. The medicament may be used for the treatment of peripheral nervous injuries and spinal cord regeneration e.g. in cases of paraplegia.

### REFERENCES TO RETINOIC ACID SECTION

1. Benbrook, D.; Lernhardt, E.; Pfahl, M. : A new retinoic acid receptor identified from a hepatocellular carcinoma. Nature 333: 669-672, 1988.
2. Brand, N.; Petkovich, M.; Krust, A.; Chambon, P.; de The, H.; Marchio, A.; Tiollais, P.; Dejean, A. : Identification of a second human retinoic acid receptor. Nature 332: 850-853, 1988.
3. Dejean, A.; Bougueleret, L.; Grzeschik, K.-H.; Tiollais, P. : Hepatitis B virus DNA integration in a sequence homologous to v-erb-A and steroid receptor genes in a hepatocellular carcinoma. Nature 322: 70-72, 1986.
4. de The, H.; del Mar Vivanco-Ruiz, M.; Tiollais, P.; Stunnenberg, H.; Dejean, A. Identification of a retinoic acid responsive element in the retinoic acid receptor beta gene. Nature 343: 177-180, 1990.
5. de The, H.; Marchio, A.; Tiollais, P.; Dejean, A. : A novel steroid thyroid hormone receptor-related gene inappropriately expressed in human hepatocellular carcinoma. Nature 330: 667-670, 1987.
6. Kreczel, W.; Ghyselinck, N.; Samad, T. A.; Dupe, V.; Kastner, P.; Borrelli, E.; Chambon, P. : Impaired locomotion and dopamine signaling in retinoid receptor mutant mice. Science 279: 863-867, 1998.
7. Lotan, R.; Xu, X.-C.; Lippman, S. M.; Ro, J. Y.; Lee, J. S.; Lee, J. J.; Hong, W. K. : Suppression of retinoic acid receptor-beta in premalignant oral lesions and its up-regulation by isotretinoin. New Eng. J. Med. 332: 1405-1410, 1995.
8. Mattei, M.-G.; de The, H.; Mattei, J.-F.; Marchio, A.; Tiollais, P.; Dejean, A.: Assignment of the human hap retinoic acid receptor RAR-beta gene to the p24 band of chromosome 3. Hum. Genet. 80: 189-190, 1988.
9. Mattei, M.-G.; Riviere, M.; Krust, A.; Ingvarsson, S.; Vennstrom, B.; Islam, M. Q.; Levan, G.; Kautner, P.; Zelent, A.; Chambon, P.; Szpirer, J.; Szpirer, C. : Chromosomal assignment of retinoic acid receptor (RAR) genes in the human, mouse, and rat genomes. Genomics 10: 1061-1069, 1991.
10. Nadeau, J. H.; Compton, J. G.; Giguere, V.; Rossant, J.; Varmuza, S. : Close linkage of retinoic acid receptor genes with homeobox- and keratin-encoding genes on paralogous segments of mouse chromosomes 11 and 15. Mammalian Genome 3: 202-208, 1992.
11. Samad, A.; Kreczel, W.; Chambon, P.; Borrelli, E. : Regulation of dopaminergic pathways by retinoids: activation of the D2 receptor promoter by members of the retinoic acid receptor-retinoid X receptor family. Proc. Nat. Acad. Sci. 94: 14349-14354, 1997.

### References to Example 1

1. Lindsay, R. *J*. *Neurosci.* **8**, 2394-2405 (1988).
2. Quinn, S. D. P. & De Boni, U*. In Vitro Cell. Dev. Biol.* **27A**, 55-62 (1991).
3. Haskell, B. E., Stach, R. W., Werrbach-Perez, K. & Perez-Polo, J. R. *Cell Tissue Res.* **247**, 67-73 (1987).
4. Rodriguez-Tebar, A. & Rohrer, H. *Development* **112**, 813-820 (1991).
5. Wion, D., Houlgatte, R., Barbot, N., Barrand. P., Dicou, E. & Brachet, P. *Biochem. Biophys. Res. Comm.* **149**, 510-514 (1987).
6. Kastner, P., Chambon, P. & Leid, M. in *Vitamin A in Health and Disease* (ed. Blomhoff, R.) 189-238 (Dekker, New York, 1994).
7. Kliewer, S. A., Umesono, K., Evans, R. M. & Mangelsdorf, D. J. in *Vitamin A in Health and Disease* (ed. Blomhoff, R.) 239-255. (Dekker, New York, 1994)
8. Mangelsdorf, D. J. & Evans, R. M. *Cell* **83,** 841-850 (1995).
9. Millbrandt, J. *Neuron* **1**, 183-188 (1988).
10. McCaffery, P., Lee, M.-O., Wagner, M. A., Sladek, N. E. & Drager, U. *Development* **115**, 371-382 (1992).
11. Duester, G. *Biochemistry* **35,** 12221-12227 (1996).
12. Drager, U. C. & McCaffery, P. in *Enzymology and Molecular Biology of Carbonyl Metabolism* Vol. 5 (eds. Weiner, H. *et al.)* 185-192 (Plenum, New York, 1995).
13. Plum, L. A. & Clagett-Dame, M. *Dev. Dynam.* **205,** 52-63 (1996).
14. Schnell, L., Schneider, R., Kolbeck, R., Barde, Y.-A. & Schwab, M. E. *Nature* **367,** 170-173 (1994).
15. Schatzl, H. M. *Trends Neurosci.* **18,** 463-464 (1995).
16. Maden, M., Sonneveld, E., van der Saag, P. T. & Gale, E. *Development* **125**, 4133-4144 (1998).

### REFERENCES TO EXAMPLE 2

1. David, S. & Agayo, A.J. Axonal elongation into peripheral nervous system "bridges" after central nervous system injury in adult rats. *Science* **214**, 931-933 (1981).
2. Cheng, H., Cao, Y. & Olsen, L. Spinal cord repair in adult paraplegic rats: partial restoration of hind limb function. Science **273,** 510-513 (1996).
3. Schwab, M.E. Nerve fibre regeneration after traumatic lesions of the CNS; progress and problems. *Phil. Trans. R. Soc. Lond. B* **331**, 303-306 (1991).
4. Bregman, B.S. *et al.* Recovery from spinal cord injury mediated by antibodies to neurite growth inhibitors. *Nature* **378,**498-501 (1995).
5. Schnell, L*. et al.* Neurotrophin-3 enhances sprouting of corticospinal tract during development and after adult spinal cord lesion. *Nature* **367**, 170- 173 (1994).
6. Li, Y*. et al.* Repair of adult rat corticospinal tract by transplants of olfactory ensheathing cells. *Science* **277**, 2000-2002 (1997).
7. Kobayashi, N.R. *et al.* BDNF and NT4/5 prevent atrophy of rat rubrospinal neurons after cervical axotomy, stimulate GAP-43 and Tal-tubulin mRNA expression, and promote axonal regeneration.*J Neurosci.* **17**, 9583-9595 (1997).
8 Wagner, M. *et al.* Regional differences in retinoid release from embryonic neural tissue detected by an in vitro reporter assay. *Development* **116**, 55-66 (1992).
9. Horton, C. & Maden, M. Endogenous distribution of retinoids during normal development and teratogenesis in the mouse embryo.*Dev. Dynam.* **202**, 312-323 (1995).
10. McCaffery, P. & Drager, U.C. Hot spots of retinoic acid synthesis in the developing spinal cord. *Proc. Natl. Acad. Sci. USA* **91**, 71947197 (1994).
11. McCaffery, P. & Drager, U.C. Retinoic acid synthesising enzymes in the embryopnic and adult vertebrate. In *Enzymology and Molecular Biology of Carbonyl Metabolism* **5** (H. Weiner et al. eds) pp173-183. Plenum Press, New York (1995).
12. Yamamoto, M*. et al.* Influence of the choroid plexus on cerebellar development: analysis of retinoic acid synthesis. *Dev. Brain Res.* **93**, 182-190 (1996).
13. Maden, M. *et al.* The distribution of endogenous retinoic acid in the chick embryo: implications for developmental mechanisms. *Development* **125**, 4133-4144 (1998).
14. Maden, M*. et al.* Vitamin A-deficient quail embryos have half a hindbrain and other neural defects. *Current Biol.* **6**, 417-426 (1996).
15. Maden, M. *et al.* The role of vitamin A in the development of the central nervous system. *J*. *Nutr* **128**, 471S-475S (1998).
16. Maden, M. Retinoids in neural development. In *Handbook of Experimental Pharmacology.* (H. Nau & W.S. Blaner eds.) Springer-Verlag, Heidelberg (1998) in press.
17. Maden, M. *et al.* Retinoic acid as a chemotactic molecule in neuronal development. *Int. J Devl. Neurosci.* **16,** 317322 (1998).
18. Kastner, P *et al.* Role of nuclear retinoic acid receptors in the regulation of gene expression. In *Vitamin A in Health and Disease.* (R. Blomhoff ed.) pp189-238. Marcel Dekker Inc., New York (1994).
19. Kliewer, S.A. *et al.* The retinoid X receptors: modulators of multiple hormonal signalling pathways. In *Vitamin A in Health and Disease.* (R. Blomhoff ed.) pp239-255. Marcel Dekker Inc., New York (1994).
20. Corcoran, J and Maden M. (1999). Nerve growth factor acts via retinoic acid synthesis to stimulate neurite outgrowth. *Nat. Neuroscience* **2,** 307-308.
21. Quinn, S.D.P. & De Boni, U. Enhanced neuronal regeneration by retinoic acid of murine dorsal root ganglia and of fetal murine and human spinal cord in vitro. *In vitro Cell. Dev. Biol.* **27A**, 55-62 (1991).
22. Wuarin, L. & Sidell, N. Differential susceptibilities of spinal cord neurons to retinoic acid-induced survuval and differentiation. *Dev. Biol.* **144**, 429-435 (1991).
23. Ved, H.S. & Pieringer, R.A. Regulation of neuroanl differentiation by retinoic acid alone and in cooperation with thyroid hormone or hydrocortisone. *Dev. Neurosci.* **15**, 49-53 (1993).
24. Corcoran, J. & Maden, M. Nerve growth factor acts via retinoic acid synthesis to stimulate neurite outgrowth. *Nature Neurosci.* in press (1999).
25. Caroni, P. & Schwab, M.E. Codistribution of neurite growth inhibitors and oligodendrocytes in rat CNS: appearance follows nerve fiber growth and precedes myelination. *Dev. Biol.* **136**. 287-295. (1989).
26. Notterpek, L.M. & Rome, L.H. Functional evidence for the role of axolemma in CNS myelination. *Neuron* **13**, 473-485. (1994).
27. Smith, D.S. & Skene, J.H.P. A transcription-dependent switch controls competence of adult neurons for distinct modes of axon growth. *J Neurosci.* **17**, 646-658 (1997).
28. Lim, F., Hartley, D., Starr, P., Song, S., Lang, P., Yu, L., Wang, Y.M. & Geller, A.I. Use of defective herpes-derived plasmid vectors. *Meth.Mol. Biol.* **62**, 223-22 (1997).

### REFERENCES TO EXAMPLE 3

Achkar, C. C., Dergiuni, F., Blumberg, B., Langston, A., Levin, A.A., Speck, J., Evans, R.M., Bolado, J., Nakanishi, K., Buck, J. and Gudas, L.J. (1996). 4-oxoretinol, a new natural ligand and transactivator of the retinoic acid receptors. *Proc.Natl.Acad.Sci. USA* **93**, 4879-4884.
Ang, H.L. and Duester, G. (1997). Initiation of retinoid signalling in primitive streak mouse embryos: spatiotemporal expression patterns of receptors and metabolic enzymes for ligand synthesis. *Dev. Dynam.* **208**, 536-543.
Barde, Y. -A., Edgar, D. and Thoenen, H. (1982). Purification of a new neurotrophic factor from mammalian brain. *EMBO J.* **1**, 549-553.
Buck, J., Derguini, F., Levi, E., Nakanishi, K. and Hammerling, U. (1991). Intracellular signalling by 14-hydroxy-4,14-retro-retinol.*Science* **254,** 1654-1656.
Campenot, R.B. (1977). Local control of neurite development by nerve growth factor. *Proc.Natl.Acad.Sci. USA* **74**, 4516-4519.
Crowley, C., Spencer, S.D., Nishimura, M.C., Chen, K.S., Pitts-Meek, S., Armanini, M.P., Ling, L.H., McMahon, S.B., Shelton, D.L, Levinson, A.D. and Phillips, H.S. (1994). Mice lacking nerve growth factor display perinatal loss of sensory and sympathetic neurons yet develop basal forebrain cholinergic neurons. *Cell* **76***,* 1001-1012.
Corcoran, J and Maden M. (1999). Nerve growth factor acts via retinoic acid synthesis to stimulate neurite outgrowth. *Nat. Neuroscience* **2***,* 307-308.
Drager, U.C. and McCafery, P. (1995). Retinoic acid synthesis in the developing spinal cord. In *Enzymology and Molecular Biology of Carbonyl Metabolism.* **5** (ed. H. Weiner et al.) 185-192 (Plenum Press, New York.
Ernfors, P, Lee, K, Kucera. J. and Jaenisch, R. (1994). Lack of Neurotrophin-3 leads to deficiencies in the peripheral nervous system and loss of limb proprioceptive afferents. *Cell* **77**, 503-512.
Farinas, I., Jones, K.R., Backus, C., Wang, X-Y. and Reichardt, L.F. (1994). Severe sensory and sympathetic deficits in mice lacking neurotrophin-3. *Nature* **369**, 658-661.
Godbout, R., Packer, M., Poppema, S. & Dabbath, L. (1996). Localization of cytosolic aldehyde dehydrogenase in the developing chick retina: in situ hydridisation and immunohistochemical analyses.*Dev*. *Dynam.* **205**, 319-331.
Jones, K.R, Farlinas, I, Backus , C. and Reichardt, L.F. (1994). Targeted disruption of the BDNF gene perturbs brain and sensory neuron development but not motor neuron development. *Cell* **76**, 989-999.
Klein, R., Silos-Santiago, I., Smeyne, R.J., Lira, S.A., Brambilla, R., Bryant, S., Zhang, L., Snider, W.D. and Barbacid. M. (1994). Disruption of the neurotrophin-3 receptor gene trkC eliminates 1a muscle afferents and results in abnormal movements. *Nature* **368**, 249-251.
Klein, R., Smeyne, R.J., Wurst, W., Long, L.K., Auerbach, B.A., Joyner, A.L. and Barbacid. M. (1993). Targeted disruption of the trkB neurotrophin receptor gene results in nervous system lesions and neonatal death. *Cell* **75**, 113-122.
Kastner, P., Chambon, P. and Leid, M. (1994). Role of nuclear retinoic acid receptors in the regulation of gene expression. In *Vitamin A in Health and Disease.* (R. Blomhoff ed.) pp 189-238. Marcel Dekker Inc., New York.
Kliewer, S.A., Umesono, K., Evans, R.M. and Mangelsdorf, D.J. (1994). The retinoid X receptors: modulators of multiple hormonal signalling pathways. In *Vitamin A in Health and Disease.* (R. Blomhoff ed.) pp 239-255. Marcel Dekker Inc., New York.
Leid, M., Kastner, P. and Chambon, P. (1992).Multiplicity generates diversity in the retinoic signalling pathways. Trends Biol. Sci. 17, 427-433
Levi-Montanlcini, R. The nerve growth factor: Thirty five years later. *Science* **237**, 1154-1164 (1987).
Lindsay, R. (1998). Nerve growth factors (NGF, BDNF) enhance axonal regeneration but are not required for survival of adult sensory neurons. *J. Neurosci.* **8**, 2394-2405.
Maden, M., Gale, E., Kostetskii, I. and Zile, M.(1996). Vitamin A-deficient quail embryos have half a hindbrain and other neural defects. *Current Biol.* **6,** 417-426.
Maden, M. Retinoids in neural development. In *Handbook of Experimental Pharmacology.* (H. Nau & W.S. Blaner eds.) Springer-Verlag, Heidelberg (1998) in press.
Maden, M., Gale, E. and Zile, E. (1998). The role of vitamin A in the development of the central nervous system. *J. Nutr.* **128,** 471S-475S.
Maden, M., Sonneveld, E., van der Saag, P.T. and Gale, E. (1998). The distribution of endogenous retinoic acid in the chick embryo: implications for developmental mechanisms. *Development* **125** in press.
Mangelsdorf, D.J. and Evans, R.M. (1995). The RXR heterodimers and orphan receptors. *Cell* **83**, 841-850.
Maisonpierre. P.C., Belluscio, L., Squinto, S., Ip, N.Y., Furth, M.E., Lindsay, R.M. and Yancopoulos, G.D. (1990). Neurotrophin-3: a neurotrophic factor related to NGF and BDNF. *Science* **247**, 1446-1451.
McCaffery, P. and Drager, U.C. (1994). Hot spots of retinoic acid synthesis in the developing spinal cord. *Proc. Natl. Acad. Sci. USA* **91**, 7194-7197.
McCaffery, P., Lee, M.-O., Wagner, M.A., Sladek, N.E. & Drager, U. (1992). Asymmetrical retinoic acid synthesis in the dorsoventral axis of the retina. *Development* **115**, 371-382.
McCormick, A. M., Napoli, J. L., Schnoes, H. K. & Deluca, H. F. (1978). Isolation and identification of 5,6-epoxyretinoic acid: a biologically active metabolite of retinoic acid.
*Biochemistry* **17**, 4084-4090.
Millbrandt, J. (1989). Nerve growth factor induces a gene homologous to the glucocorticoid receptor gene. *Neuron* **1**, 183-188.
Niederreither, K., McCaffery, P., Drager, U.C., Chambon, P. and Dolle, P. (1997). Restricted expression and retinoic acid-induced downregulation of the retinaldehyde dehydrogenase type 2 (RALDH-2) gene during mouse development. *Mech of dev* **62**, 67-68
Quinn, S.D.P and De Boni, U. (1991). Enhanced neuronal regeneration by retinoic acid of murine dorsal root ganglia and of fetal murine and human spinal cord in vitro. *In Vitro Cell. Dev. Biol.* **27A,** 55-62.
Smeyne, R.J., Klein, R., Schnapp, A., Long, L.K., Bryant, S., Lewin, A, Lira, S.A. and Barbacid, M. (1994). Severe sensory and sympathetic neuropathies in mice carrying a disrupted trk/NGF receptor gene. *Nature* **368**, 246-249.
Snider, W.D. (1994). Functions of the neurotrophins during nervous system development : what the knockouts are teaching us. *Cell* **77**, 627-638.
Tuttle R.& Mathew, W.D (1995). Neurotrophins affect the pattern of DRG neurite growth in a bioassay that presents a choice of CNS and PNS substrates. *Development* **121**, 1301-1309.
Wuarin, L. & Sidell, N. (1991). Differential susceptibilities of spinal cord neurons to retinoic acid-induced survival and differentiation. *Dev. Biol.* **144**, 429-435.

### SEQUENCES

RARalpha reverse primer: 535 tgtagctctctgagcactc 517
RARalpha1 forward strand primer 648 tacgccttcttctttcccc 666
RARalpha2 forward strand primer 376 cttttataaccagaaccgggc 396
RARalpha3 forward strand primer 111 caagtagaagccaggaaagtc 131
RARalpha4 forward strand primer 3 ctaagaagacccacacttctg 23
RARalpha5 forward strand primer 30 aagtgaggtgaaaactggg 48
RARalpha6 forward strand primer 42 ttcacagcctggcataac 59
RARalpha6 forward strand primer 24 gagaaggaagtgagccatc 42

RARbeta reverse strand primer 508 tctctgtgcattcctgctttg 488
RARbeta 1 forward strand primer 180 tggacacatgactcactacc 199
RARbeta 2 forward strand primer 598 atgttctgtcagtgagtccc 617
RARbeta 3 forward strand primer 457 gcatgtcagaggacaactg 475
RARbeta 4 forward strand primer 21 agcctggaaaatgccatc 38
RARGamma reverse strand primer 481 ttacagcttccttggacatgcc 460
RARGamma1 forward strand primer 119 agatgctgagccctagcttc 138
RARGamma2 forward strand primer 73 ttactacgcagagccactgg 92
RARGamma3 forward strand primer 169 ggaagatggaagagggaac 187
RARGamma4 forward strand primer 230 caaatttactgggggttgg 248
RARGamma5 forward strand primer 18 ggctggattttggattgaag 37
RARGamma6 forward strand primer 329 ttctgtcctctcactaccttgg 350
RARGamma7 forward strand primer 85 cattaccgcgagtcactaac 104
GAPDH

forward primer 37 cgtagacaaaatggtgaagg 56
   reverse primer 333 gactccacgacatactcagc 314
   RALDHII
forward primer 1190 gcttcttcattgacccac 1208
   reverse primer 1539 cttcaccgtcaggtctttac 1519
RXRalpha

forward primer 745 gcaaggaccggaatgagaac 764
   reverse primer 994 tctaggggcagctcagaaaag 974

RXRbeta

forward primer 1910 agaataaaggggtagtgaagg 1930
   reverse primer 2176 catcaatgtccccacttg 2159

RXRgamma

forward primer 713 tgccagtagtagccacgaag 732
   reverse primer 966 tgagcagttcattccaccc 948

## Claims

1. Use of retinoic acid receptor β2 (RARβ2) in the preparation of a medicament for the treatment of a neurological disorder.

2. Use of RARβ2 according to claim 1, wherein said neurological disorder comprises neurological injury.

3. Use of RARβ2 according to claim 1 or claim 2 wherein said medicament further comprises an agonist of RARβ2.

4. Use of RARβ2 according to claim 3, wherein said agonist is retinoic acid (RA) and/or CD2019.

5. A pharmaceutical composition comprising retinoic acid receptor β2 (RARβ2) in admixture with a pharmaceutically acceptable carrier, diluent or excipient; wherein the pharmaceutical composition is for use in the treatment of a neurological disorder.

6. A pharmaceutical composition according to claim 5 further comprising an agonist of RARβ2.

7. A pharmaceutical composition according to claim 6 wherein said agonist is retinoic acid (RA) and/or CD2019.

8. A use according to any of claims 1 to 4 or a composition according to any of claims 5 to 7 wherein said RARβ2 is in the form of a nucleic acid encoding same.

9. A use or composition according to claim 8 wherein said RARβ2 is in the form of an entity comprising a RARβ2 expression system.

10. A use or composition according to claim 9 wherein said RARβ2 expression system is in the form of a nucleic acid construct capable of directing the expression of at least part of a RARβ2 receptor.

11. A vector comprising a nucleic acid construct capable of directing the expression of at least part of a retinoic acid receptor β2 (RARβ2) receptor.

12. A vector according to claim 11 wherein said vector is a viral vector.

13. A vector according to claim 12 wherein said vector is a retroviral vector.

14. A vector according to claim 13 wherein said vector is a lentiviral vector.

15. A use or composition according to claim 9, wherein the RARβ2 expression system is in the form of a vector according to any of claims 11 to 14.

## Patentansprüche

1. Verwendung des Retinolsäurerezeptors β2 (RARβ2) bei der Herstellung eines Medikaments zur Behandlung einer neurologischen Störung.

2. Verwendung von RARβ2 nach Anspruch 1, wobei die neurologische Störung eine neurologische Verletzung umfaßt.

3. Verwendung von RARβ2 nach Anspruch 1 oder Anspruch 2, wobei das Medikament weiterhin einen Agonisten von RARβ2 umfaßt.

4. Verwendung von RARβ2 nach Anspruch 3, wobei der Agonist Retinolsäure (RA) und/oder CD2019 ist.

5. Pharmazeutische Zusammensetzung, welche den Retinolsäurerezeptor β2 (RARβ2) im Gemisch mit einem pharmazeutisch verträglichen Träger, Verdünnungsmittel oder Hilfsstoff umfaßt, wobei die pharmazeutische Zusammensetzung für die Verwendung bei der Behandlung einer neurologischen Störung bestimmt ist.

6. Pharmazeutische Zusammensetzung nach Anspruch 5, welche weiterhin einen Agonisten von RARβ2 umfaßt.

7. Pharmazeutische Zusammensetzung nach Anspruch 6, wobei der Agonist Retinolsäure (RA) und/oder CD2019 ist.

8. Verwendung nach einem der Ansprüche 1 bis 4 oder Zusammensetzung nach einem der Ansprüche 5 bis 7, wobei der RARβ2 in der Form einer diesen codierenden Nukleinsäure vorliegt.

9. Verwendung oder Zusammensetzung nach Anspruch 8, wobei der RARβ2 in der Form einer ein RARβ2-Expressionssystem umfassenden Einheit vorliegt.

10. Verwendung oder Zusammensetzung nach Anspruch 9, wobei das RARβ2-Expressionssystem in der Form eines Nukleinsäurekonstrukts vorliegt, welches die Expression wenigstens eines Teils eines RARβ2-Rezeptors steuern kann.

11. Vektor, welcher ein Nukleinsäurekonstrukt umfaßt, das die Expression wenigstens eines Teils eines Retinolsäurerezeptor β2- (RARβ2-) Rezeptors steuern kann.

12. Vektor nach Anspruch 11, wobei der Vektor ein viraler Vektor ist.

13. Vektor nach Anspruch 12, wobei der Vektor ein retroviraler Vektor ist.

14. Vektor nach Anspruch 13, wobei der Vektor ein lentiviraler Vektor ist.

15. Verwendung oder Zusammensetzung nach Anspruch 9, wobei das RARβ2-Expressionssystem in der Form eines Vektors nach einem der Ansprüche 11 bis 14 vorliegt.

## Revendications

1. Utilisation du récepteur d'acide rétinoïque β2 (RARβ2) dans la préparation d'un médicament destiné au traitement d'un trouble neurologique.

2. Utilisation de RARβ2 suivant la revendication 1, dans laquelle ledit trouble neurologique comprend une lésion neurologique.

3. Utilisation de RARβ2 suivant la revendication 1 ou la revendication 2, dans laquelle ledit médicament comprend en outre un agoniste de RARβ2.

4. Utilisation de RARβ2 suivant la revendication 3, dans laquelle ledit agoniste est l'acide rétinoïque (RA) et/ou le CD2019.

5. Composition pharmaceutique comprenant le récepteur d'acide rétinoïque β2 (RARβ2) en mélange avec un support, diluant ou excipient pharmaceutiquement acceptable ; ladite composition pharmaceutique étant destinée à être utilisée dans le traitement d'un trouble neurologique.

6. Composition pharmaceutique suivant la revendication 5, comprenant en outre un agoniste de RARβ2.

7. Composition pharmaceutique suivant la revendication 6, dans laquelle ledit agoniste est l'acide rétinoique (RA) et/ou le CD2019.

8. Utilisation suivant l'une quelconque des revendications 1 à 4 ou composition suivant l'une quelconque des revendications 5 à 7, dans laquelle ledit RARβ2 est sous forme d'un acide nucléique codant pour celui-ci.

9. Utilisation ou composition suivant la revendication 8, dans laquelle ledit RARβ2 est sous forme d'une entité comprenant un système d'expression de RARβ2.

10. Utilisation ou composition suivant la revendication 9, dans laquelle ledit système d'expression de RARβ2 est sous forme d'un produit d'assemblage d'acide nucléique capable de diriger l'expression d'au moins une partie d'un récepteur RARβ2.

11. Vecteur comprenant un produit d'assemblage d'acide nucléique capable de diriger l'expression d'au moins une partie d'un récepteur d'acide rétinoïque β2 (RARβ2).

12. Vecteur suivant la revendication 11, ledit vecteur étant un vecteur viral.

13. Vecteur suivant la revendication 12, ledit vecteur étant un vecteur rétroviral.

14. Vecteur suivant la revendication 13, ledit vecteur étant un vecteur lentiviral.

15. Utilisation ou composition suivant la revendication 9, dans laquelle le système d'expression de RARβ2 est sous forme d'un vecteur suivant l'une quelconque des revendications 11 à 14.
